# EUROPEAN PATENT SPECIFICATION

(11) **EP 1 599 597 B1**
(45) Date of publication and mention of the grant of the patent: **04.08.2010**
(21) Application number: 04711880.7
(22) Date of filing: 17.02.2004
(51) Int. Cl.: C12M 3/00, G01N 33/543, G01N 33/548

(54) **TRANSLUCENT SOLID MATRIX ASSAY DEVICE FOR MICROARRAY ANALYSIS**
LICHTDURCHLÄSSIGE FESTMATRIX-PRÜFVORRICHTUNG ZUR MIKROARRAY-ANALYSE
DISPOSITIF DE DOSAGE AVEC MATRICE SOLIDE TRANSLUCIDE DESTINE A UNE ANALYSE MICRORESEAU

(30) Priority: 24.02.2003 US 373546; 24.02.2003 US 373408; 29.01.2004 US 540720 P
(43) Date of publication of application: 30.11.2005
(73) Proprietor: Pritest, Inc., Redmond, WA 98052 (US)
(72) Inventor: GREEN, Lawrence, R., Tacoma, WA 98498 (US)
(74) Representative: Evens, Paul Jonathan
(86) International application number: PCT/US2004/004675
(87) International publication number: WO 2004/076678

(56) References cited:
- EP-A- 0 366 241
- WO-A-01/57501
- WO-A-02/083918
- WO-A-02/085926
- WO-A-02/093144
- US-A- 5 831 012
- US-A1- 2002 015 958
- US-A1- 2003 228 637
- US-B1- 6 399 295
- SHEKARCHI I C ET AL: "MICRO STICKS AS SOLID PHASE CARRIERS FOR ENZYME LINKED IMMUNO SORBENT ASSAYS" JOURNAL OF CLINICAL MICROBIOLOGY, vol. 16, no. 6, 1982, pages 1012-1018, XP002279132 ISSN: 0095-1137

## Description

### BACKGROUND OF THE INVENTION

### Field of the Invention

The present invention relates to the field of microarray analysis. More particularly, the present invention concerns methods, and apparatus relating to translucent, solid, matrix assay devices for microarray analysis. In certain embodiments of the invention, the microarray may be a reconfigurable microarray.

### Description of Related Art

Detection of trace amounts of various analytes, such as proteins, nucleic acids, lipids, metabolites, toxins, wastes, contaminants, poisons, hormones, pharmaceuticals, drugs, explosives, pathogens, biohazards, hazardous chemicals, viruses, bacteria, cells, diseased cells, etc. is extremely important for many applications in epidemiology, public health, biowarfare, environmental health and safety, forensics, disease detection and treatment and numerous other field. Bioanalytes in particular pose numerous problems in analyte detection- Many bioanalytes, such as protein variants, may be structurally similar to other related compounds, resulting in cross-reactivity and false positive detection. Other bioanalytes may be relatively unreactive to detection reagents, causing low detection sensitivity and false negative detection.

Numerous methods for detection of proteins and other bioanalytes have been developed, primarily based on some type of immunological assay. Western blots, ELISA assays, sandwich immunoassays, immunoadsorption, *etc.* all rely upon a binding interaction between an antibody or antibody fragment, such as a monoclonal or polyclonal antibody, and an analyte to be detected. The antibody-analyte complex is then detected, often by addition of a labeled secondary antibody. One format developed for bioanalyte detection has involved the use of microarrays of use for simultaneous analysis of multiple analytes on a single chip format.

Microarray analysis involves the attachment of capture molecules, such as antibodies or oligonucleotides, to a solid matrix. Typically, the array is designed so that capture molecules specific for particular target analytes are attached to identifiable locations on the matrix. After exposure to a sample suspected of containing one or more target analytes, the matrix is analyzed to determine if substances in the sample bind to the capture molecules at one or more locations on the array.

Two-dimensional microarrays have proven useful for a wide range of applications, such as genomic research. Arrays of oligonucleotide probes may be used to determine the match or mismatch for a given sample of DNA or RNA, as in the detection of disease-associated single nucleotide polymorphisms (SNPs). Gene expression-profiling with microarrays containing probes against target gene mRNAs has been used to identify genes that are up- or down-regulated in response to disease, drug treatment, developmental stage and other conditions. Microarrays have also been of use for applications in protein research. However, proteins are more difficult to attach to a solid matrix and far more complex than oligonucleotides. Thus, techniques for use with protein (antibody) microarrays often require modifications compared to the more simple nucleic acid microarrays. (See, *e.g.,* Constans, *The Scientist,* 16:28, 2002.)

Many clinical diagnostic devices have been built around microarray platforms incorporating an appropriate solid matrix. These often contain capture molecules that have been printed or otherwise permanently affixed to the matrix. One of the problems with such fixed arrays is that they are static. Once an array has been printed, it cannot be changed or adapted to conduct any tests other than the ones that it was originally designed for. A reconfigurable microarray would be very advantageous in allowing flexibility of use.

Existing microarrays face additional problems. For example, the type of solid matrix used may affect the results obtained, depending on the method of analysis and the materials used. Most microarrays are produced using covalent, electrostatic or hydrophobic binding to attach capture probes to the surface of a solid matrix. The capture probes remain attached to the surface during sample analysis. Bound target molecules may be detected in a variety of ways. Most commonly, one or more fluorophore tags are attached to the target molecules or cells that are to be bound by a capture molecule. Once binding is complete the tags may be spectrophotometrically detected. Scanners, CCD cameras or similar detectors may be used to determine the location and signal intensity of fluorescent tags bound to matrix arrays.

The amount of probe material that can be affixed on a matrix surface depends on the composition of the solid matrix. If insufficient amounts of probe are affixed to the matrix, the resulting fluorescent signal will be so weak that it cannot be detected even if the probe captures a tagged target molecule. It is also not sufficient to bind high concentrations of probe molecules to the surface of a solid matrix, if the matrix does not provide sufficient conformational or steric freedom to allow probes to bind to target molecules.

The solid matrix must also preserve the functional activity of the probe. Proteins, such as antibodies, attached to a solid matrix may undergo denaturation over time, rendering antibodies inactive or enzymes dysfunctional. In such cases, the signal strength (and the amount of target protein identified in a sample) may vary by the length of time following matrix array manufacture. Although such time-dependent processes may be compensated for in part by the use of external standard proteins, the denaturation rates for different antibodies or enzymes affixed to the same matrix may not be identical.

Other characteristics of the solid matrix used for 2D arrays may also be important. For example, the opacity of the solid matrix may render it useless for certain kinds of analysis. Opaque materials only allow sample analysis to occur on the same side of the solid matrix as the probe array. This prevents the use of see-through optics that detect light from the opposite side of the matrix. For example, a matrix array may be opposed to a fluidic cube or other fluidic device, with probe molecules attached to the array within a cavity formed by the fluidics cube. Detection of real-time binding of target molecules to the probes would be greatly facilitated if emitted light could be detected from the opposite surface of the array. This is not feasible if the array is opaque to the emitted light. A need exists for a translucent solid matrix that could be used with a variety of optical detection systems. Such translucent matrix materials should also allow for binding of high concentrations of probe molecules, while maintaining probe molecules in an active state.

Microarrays may be used in combination with optical image sensors for detection of analytes. Among possible analytic uses for image sensors is the evaluation and characterization of light interactions between one or more substances on a surface or in a reaction vessel. Light emitted by the object of interest or absorbed by the object when a beam of light passes through it may be detected and quantified.

Most modern light detectors are designed to capture a spectral signal by presenting a two-dimensional array of sensitive photodiodes towards a target. The photodiodes are designed to produce current when exposed to light, and the resulting current may be analyzed in various ways. Modem sensors convert the analog photodiode signal to a digital signal format that may then be stored and processed for later analysis.

Image sensors are often sensitive and responsive, acting to minimize background noise and interference. Image sensors are capable of accurately recording data regarding light striking the photodiode array of the sensor. High-resolution digital pictures may be produced pixel by pixel with an appropriate source of light, an optical system, an image sensor, and a computer. Using such a system, photographic pictures may be obtained in either monochromatic or color formats.

However, a photodiode will produce an analog output signal that correlates with the energy striking the photodiode array only in special circumstances, such as when the target is illuminated by monochromatic light at a particular wavelength. Even though the output signal of a photodiode is essentially linear with respect to the illumination applied to the photodiode, the signal value for a pixel does not generally correlate accurately with the photon flux. This is because the quantum efficiency (QE) for converting the photon flux to a photodiode electrical energy varies with certain factors. In addition, in most cases more than a single wavelength of light will strike a photodiode.

Every photodiode has a certain QE factor that will vary with factors such as wavelength and temperature- Photon flux represents the electromagnetic energy striking the surface of a two-dimensional array, and the QE represents the capability of the photodiode to convert that energy into electrical energy. QE is usually expressed as a percentage of the energy flux, equaling some percentage less than 100 percent. Because QE varies greatly with the wavelength of light illuminating a photodiode, comparisons of a signal at one wavelength to that at another are difficult to interpret unless the QE factors are known for all wavelengths that apply.

Further, most image sensors are designed by manufacturers to produce images that approximate the equivalent of what would be seen on a film or by the human eye. Manufacturers are interested in reproducing "life-like" pictures and colors. Manufacturers may provide access to the raw digital information for every pixel, but image sensors generally process that information before it is available for analysis to better render the "life-like" colors and intensities that represent human visual expectations.

For these reasons, the data produced by an image sensor generally does not directly relate to the photon flux that impinges upon the photodiode array of the sensor. This factor limits the usefulness of image sensors for analytic purposes.

US 6 921 637 discloses a liquid composition including a colloidal suspension of nitrocellulose particles used to form a microporous matrix.

### SUMMARY OF THE INVENTION

Aspects of the present invention are defined in the appended independent claims. Embodiments of the invention are defined in the appended dependent claims.

In certain embodiments of the invention, the matrix arrays are microarrays, with binding molecule spots of between 1 µm and 999 µm in size. In particular embodiments, the spots may be between 100 and 500 µm in size. More particularly, the spots may be about 300 µm in size. In specific embodiments, the microarrays may be reconfigurable.

The present invention provides a method of making translucent matrix arrays characterised by
a) in a first step coating a substrate with a translucent, optically clear, layer of colloidal nitrocellulose, wherein the colloidal nitrocellulose is suspended in either amyl acetate
   or other volatile organic solvents before application to the substrate which is in the form of a glass or
   other translucent slide,
   and
b) in a second step attaching one or more first probes to the translucent, optically clear, colloidal nitrocellulose layer, wherein the translucent optically clear nitrocellulose layer
   has a protein binding capacity of 100-200 µg/cm2.

The present invention further provides an optical assay device obtainable by the aforementioned method
comprising:
a translucent, nylon slide;
a translucent, optically clear, colloidal nitrocellulose layer attached to the upper surface of the slide; and
a multiplicity of probes bound to the upper surface of the translucent, optically clear, colloidal nitrocellulose layer, wherein the translucent, optically clear, colloidal nitrocellulose layer has a binding capacity of 100-200 µg/cm2.

In various embodiments of the invention, the solid matrix arrays may exhibit one or more of the following characteristics: [1] inexpensive to produce; [2] long term stability (retain characteristic features over time); [3] ease of manufacture; [4] reproducible target molecule detection and/or quantification between lots; [5] bind high levels of probes; [6] do not interfere with probe functionality (*e.g*., binding affinity for target molecules); [7] do not interfere with probe-target molecule interaction. Such characteristics of the disclosed solid matrix arrays are advantageous compared to previously known solid matrix arrays.

Such translucent matrices are ideally suited for 2D microarray analysis. The translucent nitrocellulose matrix preserves protein (antibody) functionality and exhibits many of the characteristics listed above, such as high binding capacity. The translucent nitrocellulose solid matrix is suitable for genomic and proteomic work and can be used in a variety of diagnostic formats. Because it is translucent, it is suitable for use with see-through optics that detect light from the opposite side of the matrix from the capture molecules. A non-limiting example of a fluidics cube type biosensor of use with the claimed methods and compositions is disclosed in U.S. Patent 6767733.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

**Fig. 1** shows an exemplary 96 Well Microplate Reagent and Footpad™ Format

**FIG. 2** illustrates an exemplary Footpad, showing an array with index spot and positioning in a reservoir with an inverted eppendorf pipet tip.

**FIG. 3** illustrates an exemplary method of use of a TOAD™ apparatus with a translucent nitrocellulose-coated slide (GRABBER™).

**FIG. 4** illustrates an illustrative example of a Total Optical Assay Device (TOAD™).

**FIG. 5** shows an exemplary kit for use with a TOAD™. In this non-limiting example, GRABBER™ slides are included for analyte detection. Alternative embodiments of kits may comprise 96 well microtiter plates

**FIG. 6** illustrates an exemplary set of GRABBER™ slides, along with an incubation sleeve with four reaction chambers.

**FIG. 7** shows a schematic diagram, illustrating the components of an exemplary TOAD™ apparatus.

**FIG. 8** shows a schematic of the upper surface of a TOAD™ apparatus with the casing 810 removed to show the stage 820.

**FIG. 9** shows a non-limiting exemplary embodiment of an any of Footpads™ 910 contained in a Footpad™ holder 920.

**FIG. 10** shows a side view of an array of Footpads™ 910 in a holder 920.

**FIG. 11** shows a bottom view of at array of Footpads™ 910 in a holder 920.

### DESCRIPTION OF ILLUSTRATIVE EMBODIMENTS

### Definitions

Terms that are not otherwise defined herein are used in accordance with their plain and ordinary meaning.

As used herein, "a" or "an" may mean one or more than one of an item.

As used herein, the terms "analyte," "target" and "target analyte" mean any compound, molecule or aggregate of interest for detection. Non-limiting examples of analytes include a protein, peptide, carbohydrate, polysaccharide, glycoprotein, lipid, hormone, growth factor, cytokine, receptor, antigen, allergen, antibody, substrate, metabolite, cofactor, inhibitor, drug, pharmaceutical, nutrient, toxin, poison, biowarfare agent, biohazardous agent, infectious agent, prion, vitamin, waste product, or any other molecule or atom, without limitation as to size. "Analytes" are not limited to single molecules or atoms, but may also comprise complex aggregates, such as a virus, bacterium, *Salmonella, Streptococcus, Legionella, E. coli, Giardia, Cryptosporidium, Rickettsia,* spore, mold, yeast, algae, amoebae, dinoflagellate, unicellular organism, pathogen or cell. In certain embodiments, cells exhibiting a particular characteristic or disease state, such as a cancer cell, may be target analytes. Virtually any chemical or biological compound, molecule or aggregate could be a target analyte. "Bioanalytes" are "analytes" that are associated with, part of, contained within, incorporated into, metabolites of and/or products of biological systems (e.g., organisms, cells, bacteria, viruses, etc.)

As used herein, "capture molecule" or "probe" refers to a molecule or aggregate that has binding affinity for one or more targets. Within the scope of the present invention virtually any molecule or aggregate that has a binding affinity for some target of interest may be a "probe." "Probes" include, but are not limited to, polyclonal antibodies, monoclonal antibodies, antibody fragments, FAb fragments, humanized antibodies, single-chain antibodies, chimeric antibodies, affibodies, oligonucleotides, polynucleotides, nucleic acids, aptamers, nucleic acid ligands and any other known ligand that can bind to at least one target molecule. In certain embodiments, the capture molecule is specific for binding to a single target, although in other embodiments the capture molecule may bind to multiple targets that exhibit similar structures or binding domains. In preferred embodiments, the capture molecule is an antibody. Primary antibodies for detection of one or more targets of interest may be provided on a pre-spotted slide or may be selected by the user to load onto a slide or other array.

The terms "detection" and "detecting" are used herein to refer to an assay or procedure that is indicative of the presence of one or more specific analytes in a sample, or that predicts a disease state or a medical or environmental condition associated with the presence of one or more specific analytes in a sample. It will be appreciated by those of skill in the art that all assays exhibit a certain level of false positives and false negatives. Even where a positive result in an assay is not invariably associated with the presence of a target analyte, the result is of use as it indicates the need for more careful monitoring of an individual, a population, or an environmental site. An assay is diagnostic of a disease state or a medical or environmental condition when the assay results show a statistically significant association or correlation with the ultimate manifestation of the disease or condition

In a non-limiting embodiment, "GRABBER^{™} slides" refer to nylon slides coated with a layer of translucent nitrocellulose, as discussed in more detail below. The nitrocellulose may be applied to a surface with an icon relief. Spotting may be done using a spotting template with the icon in a corner (e.g., the upper left or lower right corner) of the template and the nitrocellulose surface exposed. The invention is not limited to the illustrated preferred embodiments and the skilled artisan will realize that other types of slides, including but not limited to glass or other types of plastic slides, may be used in the practice of the claimed methods and apparatus.

A "spotting template" refers to a tool that may hold slides (e.g., up to 6 slides at a time) when applying probes to the surface of a slide. The template may be used to accurately place spots in the proper field that will be obtained in imaging. In an exemplary embodiment, slides may be provided with diamond marker points. Spotting of probes in the region between the diamond points provides for accurate imaging.

In certain embodiments, a "focus template slide" is a glass slide with a semi-translucent template grid pattern identical to the spotting template. The slide may be used on the stage of the TOAD™ to confirm that the test slide has been properly positioned for imaging. It is also useful in helping the user identify the position of a particular spot in the image.

In some embodiments, "index marking solution" refers to a ready formulated index protein with biotin affixed that when spotted on the surface of a slide, serves as a convenient position marker and positive control. The solution may be used without dilution by spotting onto the surface of the slide a column or row of spots in the same manner as is used in affixing primary probe antibodies.

An "incubation sleeve" may refer to a sleeve with a sticky surface that is applied to a nitrocellulose surface, forming an incubation chamber. In certain embodiments, there are two openings on a diagonal in the window that may be used to fill and or empty the chamber during incubation. The sleeve may be affixed to a slide after the 2D array is formed, but before blocking, and may remain in place throughout the test, even during the imaging procedure. Just before imaging, after the luminol test solution is placed in the sleeve incubator, the thin surface outer window sheet may be peeled away to present a totally clear window during imaging.

"TOAD™ bioilluminator" refers to an imaging apparatus, equipped with a stage to accommodate a prepared slide, set of microtiter wells or other device to be optically imaged. The slide or other device may be placed on the stage so that a sleeve incubator (face up) is positioned accurately in the field for imaging. This may be confirmed in advance using a focus template slide and imager. Generally when an array is spotted between the diamond points on a template, the sleeve will be centered directly above the lens in the slotted stage. The apparatus is not limited to the disclosed preferred embodiments, and other types of devices besides slides or microtiter wells may be utilized for analyte detection with a TOAD™ bioilluminator.

### Traditional Translucent Slides

Glass or plastic microscope slides have commonly been used as solid matrix supports for microarray analysis. Probe molecules have been attached to glass or plastic surfaces using cross-linking compounds. (See, *e.g*., Schena, Microarray Analysis, J. Wiley & Sons, New York, NY, 648 pp., 2002.) Probes may be printed as 2D arrays of spots, each of about 100 to 500 µm in diameter. The cross-linking compounds and any coating used to attach probes to the glass surface form a solid matrix, on top of the glass substrate. Many different kinds of cross-linkers are known, depending on the type of reactive moieties (*e.g*., sulfhydryl, amino, carboxyl, phenyl, hydroxyl, aldehyde, etc.) available on the probe molecules that can be cross-linked to the surface without affecting probe functionality (*e.g*., target molecule binding).

A problem with previous methods for probe attachment is that the capacity for attachment is limited. As probe size is increased, the number of possible binding sites for prospective target molecules is generally decreased. If the binding sites for the probe are saturated at a level below the threshold for detection, a signal will not be observed even if binding has occurred between probe and target molecule.

Attempts have been made to attach probes to the glass surface using avidin-coated slides and biotin-conjugated probe molecules. Alternatively, silanes, such as aminosilane or 3-glycidoxypropyltrimethoxysilane, have been coated onto the glass surface, with the silane moiety attached to the glass and the reactive moiety cross-linked to probe molecules. Other approaches have utilized slides coated with reactive substrates with functional aldehyde, carboxyl, epoxy, or amine groups that can form a covalent bond with the probe molecules, affixing them permanently to the glass surface.

Although these methods work moderately well for small probe molecules, they tend to work poorly for larger probe molecules (*e.g*., antibodies) where functionality (binding) may depend on probe orientation, flexibility and degree of cross-linking. Covalent attachment methods also tend to bind very little material to the matrix surface. Consequently, probe concentration is low and signal detection is difficult. Because relatively little probe is available on the surface of the 2D array, such systems show a low signal-to-noise ratio for a positive binding reaction between probe and target.

Protein or peptide target molecules are often detected using antibodies as capture molecules. Two-dimensional arrays used in clinical diagnostics or proteomics frequently utilize antibodies as probes for protein or peptide target molecules. Although antibodies tend to be highly specific for their target antigens, they are not easily attached to glass surfaces with cross-linking agents and standard methods. This is because of the limited amount of material that can be affixed to the matrix with known chemistries, resulting in weak signals generated upon target binding. Another problem is that antibody specific binding cannot be maintained without adequate hydration and support in the matrix. Thus, long term stability of antibody-coupled solid matrix arrays tends to be limited, with inconsistent results obtained depending on the age of the array.

Attempts have been made to solve this problem by creating an environment that stabilizes the protein and preserves its functional probe features. For example, Prolinx Inc. (Bothell, WA) has developed a chemical affinity system using standard glass slides with a polymer brush format affixed to their surface. The system relies upon the interaction between two synthetic small molecules that form a stable complex, phenyldiboronic acid (PDBA) and salicylhydroxamic acid (SHA). *(E.g.,* Stolowitz et al., Bioconjugate Chem. 12:229-239, 2001.) PDBA is first conjugated with protein probes. The conjugated probes then link to SHA attached to the polymer brush to form a 3D functional array. This method is limited by the amount of antibody that can be bound to the surface. More importantly, the target antigen must be sufficiently small to diffuse through the brush border in order to react with antibodies affixed to the matrix. Such methods are not suitable for identifying and/or quantifying larger targets, such as whole cells or bacteria.

### Opaque Slides

Methods to stabilize and increase the amount of probe attached to matrix arrays are highly desirable. Such methods generally lead to opaque slides, since the matrix materials used to increase probe binding and preserve stability typically involve non-translucent gels, hydro-gels, agars, and other materials coated on the glass surface. Proteins attached to such opaque matrix materials are stabilized by hydrophobic and electrostatic interactions in a three-dimensional array.

Most scanners in current use for genomic and proteomic microarrays read the slides from the same side as the bound probe and target molecules, using opaque matrix arrays. Opaque matrix-coating materials used to produce microarrays include nylon, PVDF (polyvinylidene fluoride) and nitrocellulose. Nitrocellulose, a traditional polymer substrate in use for more than 50 years, is a substrate with very attractive properties for microarray applications. (*E.g.*, Tonkinson and Stillman, Frontiers in Bioscience 7:c1-12, 2002.)

Opaque nitrocellulose has been extensively used to immobilize proteins and nucleic acids for biomolecular analysis. Nitrocellulose immobilizes molecules of interest in near quantitative fashion and allows for short and long term storage. Nitrocellulose also allows for solution phase target species to efficiently bind to immobilized capture molecules. Diagnostic devices using ELISA methods have employed nitrocellulose membranes with a lateral flow process to bind capture reagents to the membrane (Jones, IVD Technology, 5(2):32, 1999).

Traditional opaque membrane materials have a number of attractive features. They are inexpensive to construct, bind more than 100 times the amount of protein that can be bound by linker coated glass slides, and are generally easy to work with. This is particularly true for opaque nitrocellulose membranes, which have a long history of use.

Nitrocellulose is normally produced in a microporous form that may be applied to the surface of glass slides to form an opaque surface. Probes may then be attached to the opaque nitrocellulose membranes in microarrays, using standard nitrocellulose binding methods. Such slides have been used with radioactive, fluorescent and chemiluminescent detection systems (*e.g*., Brush, The Scientist 14[9]:21, 2000).

Traditional nitrocellulose membranes are also very brittle in the absence of a supporting structure or foundation, leading to frequent cracking or fragmentation. For this reason, opaque nitrocellulose has been used in a microporous form bound to plastic sheets. Such sheets are always opaque, due to the microporous form, and require a supporting structure (*e.g*. acetate or cellulose) to avoid damage during handling.

Although opaque nitrocellulose membranes exhibit high binding capacity, stability and reasonably low interference with target-capture molecule interactions, they are not suitable for use with optical detection systems designed to work from the opposite side of a matrix array.

### Translucent Nitrocellulose Slides

The methods disclosed herein may be used to produce translucent surface coatings of colloidal nitrocellulose that retain advantageous binding characteristics of opaque nitrocellulose membranes. The interaction between probe and target molecules can be observed directly on a translucent nitrocellulose solid matrix.

In some embodiments of the invention, translucent nitrocellulose matrix arrays may be used in combination with a flow cell, fluidics cube or capillary systems . In such embodiments, the translucent nitrocellulose matrix may be attached to one side of a glass or plastic slide. Probes may be attached to the nitrocellulose and the interaction between probe and target molecules observed through the glass with a sensor or camera.

The nitrocellulose material is totally translucent if formed according to the disclosed methods. Light signals may thus be observed without scatter or interference from opaque materials. This allows a greater signal-to-noise ratio and ease of detection of target molecules, compared to opaque microporous nitrocellulose matrix array. Such opaque matrix arrays can obscure portions of the light or reaction indicator species (*e.g*., dye) produced upon binding of target molecules.

Nitrocellulose in the form of a colloid in an amyl acetate solvent has been used by electron microscopists to make castings for specimens. Colloidal nitrocellulose is formed by casting as an ultra-thin film on a water surface. The film may then be picked up on a transmission electron microscopy (TEM) grid and used as a support film for TEM specimens. Because the film must be very clean and uniform, great care is exercised in its production. Colloidal nitrocellulose is readily soluble in amyl acetate. The amyl acetate is water soluble and evaporates evenly to form uniform films. It is supplied as a 1% solution of very pure nitrocellulose.

High purity nitrocellulose in EM grade amyl acetate (Collodion) may be purchased from commercial sources. The amyl acetate is purified by refluxing over calcium oxide to remove all moisture. Soluble and suspended material is removed by slow distillation. The removal of all traces of moisture from the solvent permits the formation of very strong colloidal nitrocellulose films with virtually no holes.

In an exemplary embodiment of the invention, Collodion was obtained in bulk from Ernest F Fullam, Inc. (Latham, NY) and used to manufacture high quality translucent nitrocellulose matrix arrays. An aliquot of 200 µL of 1% Collodion solution was pipetted onto the surface of freshly cleaned standard 25 x 75 mm glass slides. The Collodion was evenly spread to the edges of the glass slide surface in a dust free area. After drying for 2 hours at room temperature, the slides were heated for an additional hour or more at approximately 60°C. Dried slides were labeled and stored for production of microarrays.

When using a glass array surface, the edges of each slide were sealed with lacquer (*e.g*. nail polish) or other adhesive to prevent the ultra-thin nitrocellulose substrate from separating from the glass upon exposure to aqueous solutions. When colloidal nitrocellulose is applied to acetate film, nylon slides or other plastic surfaces, it requires no adhesive and binds avidly. Slides may be composed of almost any translucent material as long as the amyl acetate does not react with the surface to discolor it or alter its properties. Certain types of plastics become opaque when exposed to amyl acetate and are not suitable for use with that solvent system. In alternative embodiments of the invention, the colloidal nitrocellulose may be suspended in other volatile organic solvents besides amyl acetate before application to a glass or other translucent slide.

The colloidal nitrocellulose slides may be spotted with probes using any known methods for microarray production. Methods for spotting proteins, peptides, oligonucleotides and nucleic acids onto nitrocellulose surfaces are well known in the art. Antibodies and biotinylated bovine serum albumin were used to determine the colloidal nitrocellulose binding capacity. The estimated protein binding capacity for the initial glass matrix arrays was in the range of 100 to 200 µg/cm².

A CMOS imaging system that detected light emissions through the translucent nitrocellulose matrix arrays was used with a Cy5-streptavidin indicator dye. (See, *e.g*., U.S. Patent 6 767733). At a loading volume of approximately 5 nanoliters per spot, biotinylated BSA was reproducibly detected at least down to 10 to 20 picograms of protein, Optimal CMOS images were obtained using a protein concentration range of about 20 to 100 µg/mL biotinylated BSA. The CMOS imaging data was confirmed using a 24 hour colloidal gold stain (BioRad, Hercules, CA) of the spot arrayed translucent nitrocellulose slides.

The ability of the colloidal nitrocellulose matrix to maintain antibody binding activity was confirmed by spotting a variety of antibodies on the translucent surface. A concentration range of 20 to 200 µg/mL was used. Nonspecific protein binding sites on the nitrocellulose membrane were blocked with 0.1% BSA in buffer solution. The antibodies were then exposed to solutions containing the appropriate target antigen. Binding was detected using biotinylated second antibodies and Cy5-streptavidin indicator.

A 2D array spotted with 5 nanoliters per spot of primary goat anti-mouse antibody solution and was developed using a secondary mouse *Listeria* monoclonal antibody and a secondary biotinylated goat anti-mouse antibody. In this assay, the primary and secondary goat anti-mouse antibodies bound to different epitopes of the secondary mouse antibody. The two secondary antibodies were thus used to detect primary antibody bound to the array. Using a CMOS imaging system, the primary antibody could be detected down to a level of 100 picograms or less of antibody. The CMOS data was confirmed on the same slides using colloidal gold staining.

An advantage of the translucent nitrocellulose surface is that the progress of the probe binding reaction can be examined by looking through the translucent lower surface of the slide. This allows more effective probe binding to occur. The slide may also be adapted for use with a fluidic cube to mix and deliver samples to the surface. The progress of the probe-target binding reaction may also be monitored in real time through the underside of the slide.

### Reconfigurable Microarrays

In certain embodiments of the invention, reconfigurable microarrays may be produced by using small linker molecules, such as aptamers or affibodies, bound to the surface of a solid matrix. Aptamers are oligonucleotides derived by an *in vitro* evolutionary process called SELEX (*e.g.,* Brody and Gold, Molecular Biotechnology 74:5-13, 2000). Aptamers may be produced by known methods (*e.g*., U.S. Patent Nos. U.S. Pat. Nos. 5,270,163; 5,567,588; 5,670,637; 5,696,249; 5,843,653) or obtained from commercial sources *(e.g,* Somalogic, Boulder, CO). Aptamers are relatively small molecules on the order of 7 to 50 kDa. Because they are small, stable and not as easily damaged as proteins, they may be bound in higher numbers to the surface of a solid matrix. This effectively amplifies the number of probe reactive sites on the surface of an array.

Affibody® ligands (U.S. patent no. 5,831,012) are highly specific affinity proteins that may be designed and used like aptamers. Affibodies may be produced or purchased from commercial sources (Affibody AB, Bromma, Sweden). Aptamers and affibodies may be used in combination with antibodies to increase the functional avidity of translucent or non-translucent solid matrices for probe molecule binding. Increased binding in turn results in an increased signal strength, greater signal-to-noise ratio, more reproducible target molecule detection and greater sensitivity of detection.

Reconfigurable microarrays may be used in combination with two antibodies and a capture probe. The capture probe may be an affibody, aptamer or any other probe capable of binding one of the antibodies. Both antibodies should selectively bind to a target cell, molecule or antigen.

The effectiveness of binding is increased if the capture probe binds to a portion of an antibody characteristic of the IgG class. Such probes would only require a small part of the antibody structure to be present in order to react and bind to an antibody-target complex. Larger targets, such as microbes or cells are covered with numerous antigens that may form very large complexes with antibodies. However, truncated IgG antibody fragments could interact with such large targets and still bind to an aptamer or affibody probe on the slide surface..

Antibodies are most stable in solution and optimal antigen-target molecule binding occurs with antibodies in solution, not attached to a solid matrix. In preferred embodiments of the invention, the two antibodies may be allowed to bind to the target in solution. Once target-antibody complexes are formed, the complex may be exposed to aptamer or affibody probe molecules on the reconfigurable matrix array. The probes may bind to a first antibody, while the second antibody may be conjugated to a fluorescent tag or other marker. The tagged complex may then be detected on the surface of the matrix array, using optical detection or any other known detection method.

For example, an aptamer may be tailored to specifically bind to the Fc portion of mouse IgG with high affinity. Samples containing target molecules of interest may be allowed to interact in solution with a mouse antibody specific for an antigen of interest. The sample may be mixed with a different biotinylated or otherwise tagged second (non-mouse) antibody that binds to a different epitope on the same antigen. The target antigen bound to the first and second antibodies may be exposed to the aptamer microarray. The anti-mouse aptamer affixes the complex to the solid matrix. After extensive washing to remove unbound tagged antibodies, the complex containing tagged antibody that is attached to the matrix array surface may be detected.

In some embodiments of the invention, multiple analytes may be simultaneously detected on a reconfigurable microarray. Such multiplex assays require that each second antibody specific for a different target antigen be labeled with a distinguishable label. For example, three different second antibodies may be tagged with red, green or blue fluorophores. Using anti-mouse aptamers, mouse antibodies specific for three different targets may be added to a sample and mixed with the target molecules and second antibodies. After exposure to the aptamer array, the presence of each target may be determined by the presence of red, green or blue fluorophores attached to the matrix. The skilled artisan will realize that the invention is not limited to optically distinguishable fluorophore tags and that any known identifiable tag moieties, such as radioactive, fluorescent, luminescent, enzymatic, etc. may be used. The number of target analytes that may be simultaneously detected is limited only by the number of distinguishable tags that may be attached to the second antibody.

The skilled artisan will realize that many variations on this scheme may be used within the scope of the claimed methods. For example, in alternative embodiments of the invention, a first antibody may be used in conjunction with multiple tagged second antibodies, each of which binds to a different epitope of the target molecule. This may occur, for example, where the available second antibodies are polyclonal antibodies. Alternatively, use of more than one second antibody with affinity for the same antigen may improve the sensitivity of detection. In another alternative, one second antibody may bind to a class of targets (for example, all *E*. *coli* bacteria) while a second antibody binds to a specific subclass (*e.g., E. coli* strain O157:H7).

In a non-limiting example, the aptamer detection method may be used to detect microbes in a food sample. An aptamer that exhibits high affinity and specific binding for mouse IgG may be obtained. Such aptamers may be rapidly and readily obtained using SELEX. The anti-mouse IgG aptamer may be attached to a slide using standard methods, preferably with a translucent solid matrix. Non-specific binding sites on the matrix may be blocked and the slide washed before testing.

To detect *Listeria monocytogenes,* an IgG mouse anti*-Listeria m.* antibody may be incubated with a food sample of interest at an appropriate concentration (typically 1 to 50 µg/mL). A rabbit (or other non-mouse) biotinylated secondary anti*-Listeria m.* antibody (1 to 50 µg/mL) and incubated for 5 to 30 minutes. The sample with both antibodies may then be applied to the array containing anti-mouse IgG aptamers. After a short interval (approximately 15 minutes) the array may be washed so that only mouse IgG and rabbit biotinylated antibody complexed with *Listeria m.* is retained on the array. A solution of Cy5-strepavidin or other indicator applied to the surface may then reveal the presents or absence of an anti*-Listeria m.* antibody complex affixed to the surface.

A single aptamer with high selectivity for IgG mouse antibodies may be used as a universal extender to capture and detect a variety of microbes. The only requirement would be that the mouse antibody to the microbe or antigen reacts only with the target and the aptamer, while the biotinylated second antibody reacts only with the target and not with the aptamer.

Such an aptamer array is reconfigurable and is dependent only upon the nature of the solutions delivered to its surface. For example, an aptamer array that binds to mouse IgG antibodies may in principal be used to detect any target for which a mouse IgG is available. It is far more cost effective to construct such a reconfigurable array, compared to constructing a series of fixed pattern arrays, each of which may only detect a limited number of targets. Thus, the present invention provides significant cost advantages for use in proteomic and genomic work.

Although the methods disclosed in this section may be used with opaque arrays, they are most effectively used with a translucent matrix where solutions are delivered on one side and examined for reactivity from the opposite side of the array. A fluidic cube, total optical assay device (TOAD™) or the equivalent may be attached to the reactive surface to deliver fluids to various parts of the array, allowing the user to determine which test results will be obtained for a given sample.

Aptamers are more stable than antibodies and large proteins. They are also smaller and bind in higher concentrations to reactive surfaces. Thus, aptamer-basted systems extend the avidity of an array for target molecules beyond what could be achieved using larger proteins for array binding. Although the present example is presented with regard to aptamers, the skilled artisan will realize that affibodies or other small ligands could be used in the practice of the claimed methods.

### TOTAL OPTICAL ASSAY DEVICE (TOAD™)

### TOAD™ Bioilluminator

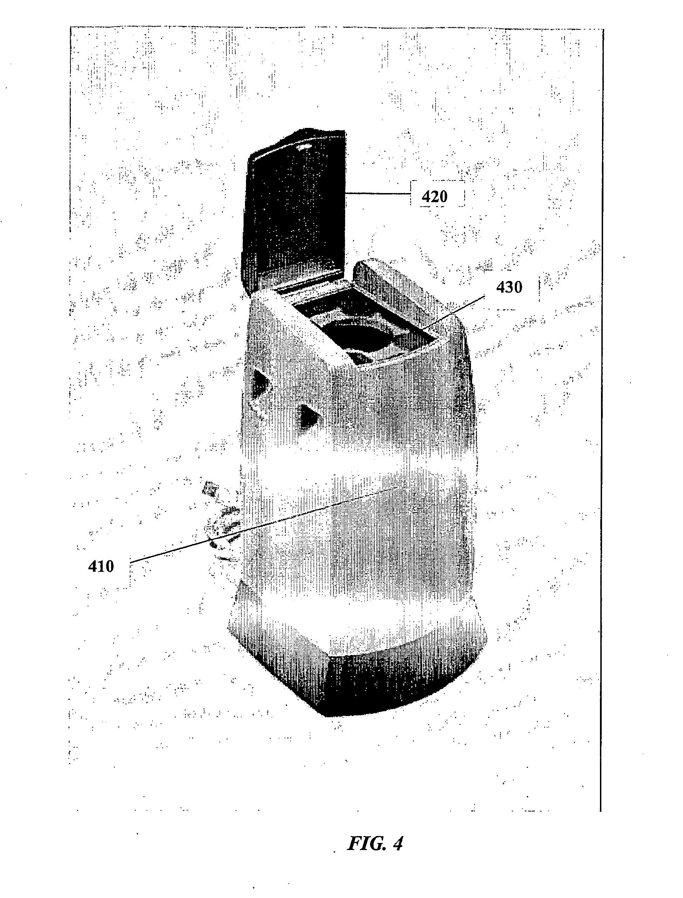

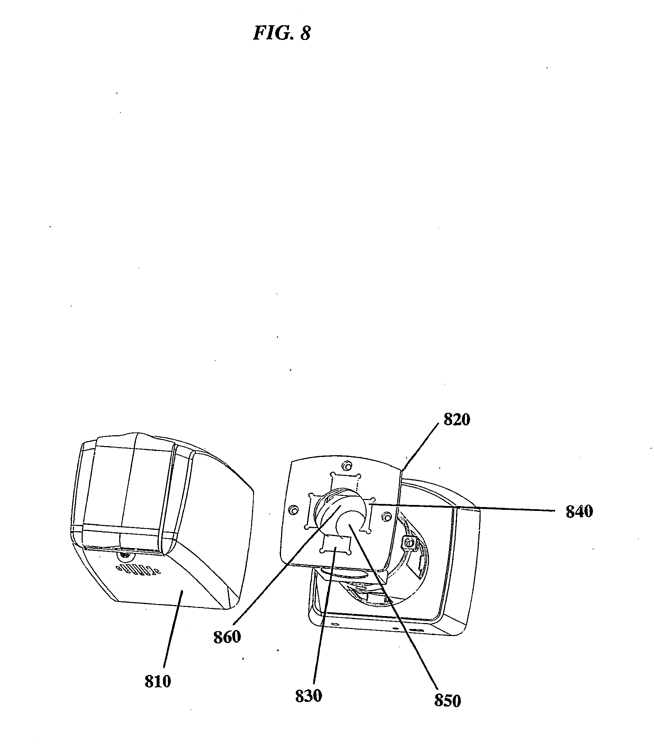

FIG. 4 illustrates a non-limiting exemplary form of a Total Optical Assay Device (TOAD™). In this example, the TOAD™ comprises a light-tight casing 410 with a hinged lid 420. When the lid 420 is closed, the casing 410 blocks external light from the interior of the TOAD™, allowing highly sensitive detection of bioluminescence or other emitted light from samples to be analyzed. The lid 420 is opened to show a stage 430. Devices to be imaged, including but not limited to GRABBER™ slides or FOOTPAD™ microtiter well devices, may be positioned on top of the stage 430. Underneath the stage is a single focusing lens (not shown) that sits on top of a CCD (charge coupled device) chip or other light sensor (not shown). The slide or microtiter wells may be positioned on the stage 430 by aligning them with corresponding shaped depressions (see FIG. 8) on the top of the stage 430. The casing 410 may comprise depressions, handles or other features to facilitate moving the TOAD™.

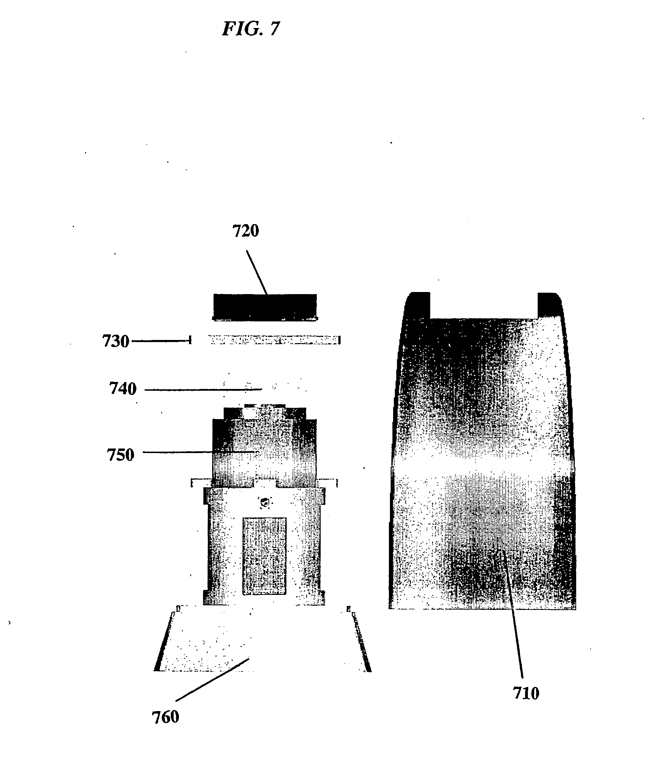

FIG. 7 illustrates a schematic view of an exemplary form of a TOAD™, with the body of the casing 710 displaced to show the internal components. The lid 720 overlies a stage 730, which is used to position slides, microtiter wells or other devices over a CCD chip located at the top of an imaging device 750. In a non-limiting example, the imaging device 750 is a QICAM 10-bit mono cooled CCD camera (Qimaging™, Burnaby, B.C., Canada, catalog #QIC-M-10-C). However, the TOAD™ is not limited to the exemplary embodiment and it is contemplated that other known types of imaging devices 750 may be utilized. A single focusing lens (not shown) is interposed between the CCD chip and the stage 730 and may be attached to the top of the imaging device 750, for example using a C-mount adaptor.

In the exemplary arrangement imaging device 750 contains, in addition to a CCD chip, a Peltier heat exchange element surrounding the CCD chip. The rest of the imaging device 750 contains electronic components for processing the signals detected by the CCD chip, along with connections for a power supply, software interface and external data port, such as a 6-pin firewire connector. The Peltier heat exchanger removes heat from the CCD camera 750 and releases it inside the casing 710. In order to prevent the TOAD™ from overheating, a separate fan driven cooling device 740 is positioned adjacent to the top of the imaging device 750. The cooling device 740 drives hot air out of the casing through a light-tight vent at the back of the casing 710 (not shown) and circulates cool air through the casing 710 interior. During standard operation, the cooling device 740 operates efficiently enough that the CCD camera 750 may be operated continuously without overheating. Overheating of the CCD camera 750 results in substantial increase in background noise, producing random bursts of apparent light detection throughout the optical field of the CCD chip.

The imaging device 750 rests on top of a base 760 that holds the imaging device 750 in position relative to the stage 730. The bottom of the base 760 is open to allow access to on on-off switch at the bottom of the imaging device 750 and connections to power supplies (*e.g*. 110 volt AC electrical cord), firewire and any other external connections. A non-limiting list of components that may be incorporated into a TOAD™, including exemplary forms and sources of such components, is included in Table 6.

FIG. 8 is a schematic diagram showing the top view of an exemplary form of a TOAD™, with the casing 810 removed to show the upper surface of the stage 820. Indentations 830, 840 are shown in the top of the stage 820, arranged to allow positioning of slides, microtiter wells or other devices over the CCD chip. A hole 860 in the center of the stage 820 allows for light transmission through the slide, microtiter well or other device and detection by the underlying CCD chip 850. It will be apparent to the skilled artisan that the use of transparent or translucent devices, as discussed in more detail below, facilitates the use of a TOAD™ apparatus with an optical detector located below the device to be optically read.

The exemplary arrangement discussed above is designed for optical detection with samples that spontaneously emit light, for example using any known chemiluminescent or bioluminescent detection system, such as the luminol system disclosed below. In this case, no external source of excitatory light is needed to detect bound analytes. The skilled artisan will realize that alternative systems may be utilized, such as fluorescently tagged detection molecules that bind to target analytes. Such systems are well known in the art. The use of a fluorescent detection system would necessitate additional components, such as an excitatory light source (*e.g*. laser, photodiode array, *etc*.), cutoff filters to screen the photodetector from excitatory light, and other such known components for fluorescent detection systems. The illustrative arrangement discussed above possesses the advantages of simplicity of construction and use, low cost, and sensitive detection of target analytes with minimal background noise.

### Footpads™

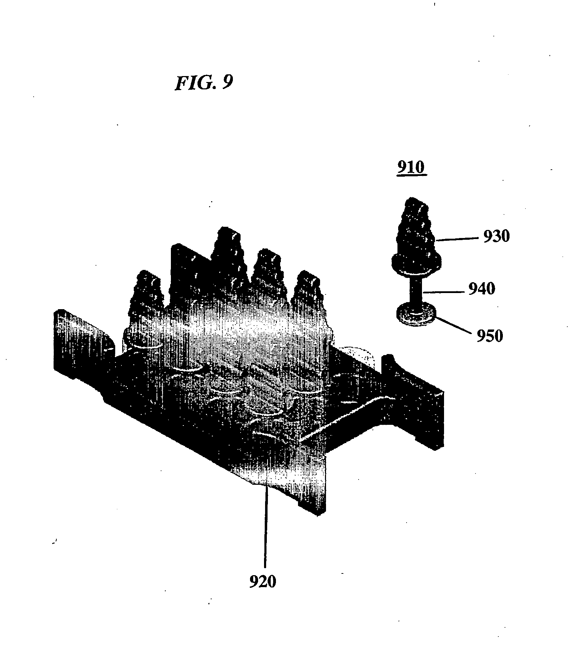

Devices called Footpads™ may come in a variety of designs, an exemplary form of which is shown in FIG. 9. FIG. 9 illustrates an array of 12 Footpads™ 910 contained in a Footpad holder 920. A single Footpads™ 910 has been removed from the array to illustrate its structure. The top end 930 of the Footpads™ 910 may be designed for ease of handling, for example with a tapered, ridged shape as shown. A stem 940 may connect the handle 930 to the bottom 950 of the Footpad™ 910. The lower surface of the Footpads™ 910 may be coated with translucent nitrocellulose, rayon, cellophane or other known materials that may bind to probes, analytes or other ligands, as discussed below. The Footpad™ 910 may come prespotted with various probes, calibration spots and other spots, as discussed below. Alternatively the user may load any molecule of interest, such as antibodies or other probes, on the lower surface 950 of the Footpad™ 910.

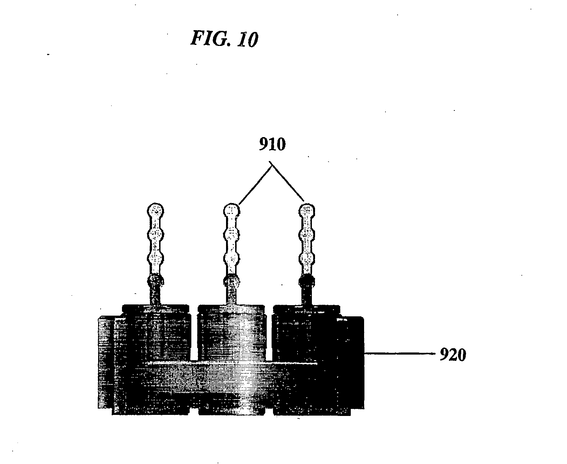

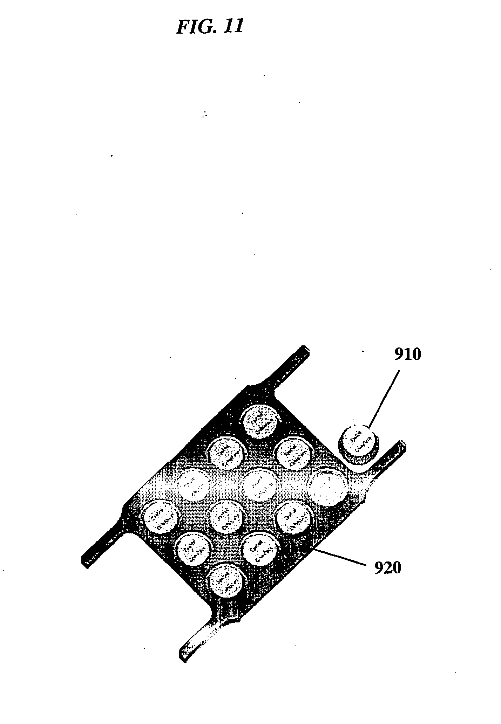

A side view of an array of Footpads™ 910 inserted into a Footpad™ holder 920 is shown in FIG. 10. When inserted fully into the holder 920, the Footpads™ 910 are contained in wells that hold the Footpads™ 910 firmly in place. The spacing of Footpads™ 910 in the holder 920 is designed so that the entire array may be inserted into the wells of a standard 96 well microtiter plate. FIG. 11 shows that the bottom of the Footpad™ holder 920 is open, with space around the rim of the Footpad™ 910 to accommodate the well rims on a microtiter plate. As discussed below, a microtiter plate may be preloaded with reagents and the holder 920 with Footpads™ 910 sequentially immersed in consecutive sets of corresponding microtiter wells. Thus, twelve or more Footpads™ 910 may be exposed to appropriate reagents simultaneously. The holder 920 and Footpads™ 910 may be transferred manually between different sets of wells of a microtiter plate. Alternatively, the holder 920 and/or Footpads™ 910 may be designed to be handled robotically.

### Footpad™ Kits

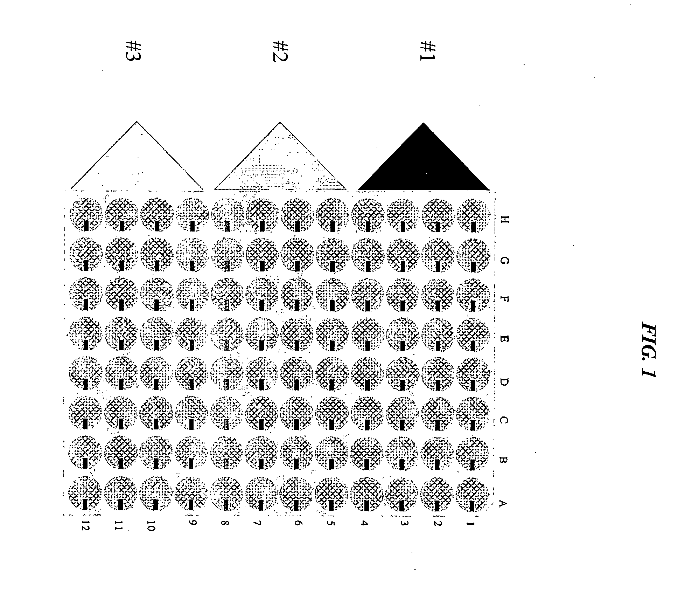

In certain arrangements, a standard 96 well plate with a flat bottom optical surface may be used with pre-loaded reagents and Footpads™ on a bioluminescent TOAD^{™} detector. A packaged kit may include a labeled, foil sealed 96 well plate with 12 TOAD^{™} Footpads™ and reagents. Super signal reagents Luminol and peroxide may be mixed immediately before a test and added to the "optical" reservoir H before beginning the test (FIG. 1). The test sample may be placed in the A reservoir (FIG. 1). As indicated in FIG. 1, in certain arrangements, sample is placed in column A wells, blocking solution (100 µL) in column B wells, wash solutions (100 µL) in columns C, E and G wells, antibody cocktail (200 µL) in column D wells, HRP-SA solution (100 µL) in column F wells and luminol solution (100 µL) in column H wells of a 96 well microtiter plate format. The skilled artisan will realize that the disclosed methods and apparatus are not limited to a 96-well format and other formats may be used. Using the 12 Footpad™ holder disclosed above, each column comprising 4 Footpads™ may be sequentially exposed to the solutions in columns A-H of FIG. 1, starting with the first column of 4 Footpads™ immersed in the column A (sample) wells and moving the holder 1 column at a time through the B-H wells. Where solutions are present in less than all 8 columns of the microtiter plate, as discussed below, the Footpad™ holder may be manipulated accordingly.

Luminol-peroxide reagent is a solution containing luminol and peroxide that is prepared and used on the same day tests are initiated. Luminol in the presence of HRP (horse radish peroxidase) and peroxide forms an unstable intermediate that produces light (see below). In the process it is consumed. Because it is in abundant excess relative to the amount of HRP trapped in a microspot on the surface of a slide or in a microplate well, light emission may be sustained for several hours, The Luminol and peroxide may be dispensed and mixed in equal volumes. The solution is then diluted using dilution buffer equivalent to the total volume in the first mixture (50% dilution). For example, if 1 ml of Luminol is combined with 1 ml of peroxide, then 2 ml of dilution buffer may be used to dilute the reagent to produce 4 mL of solution for testing.

A stage may be fitted to the TOAD™ detector to accommodate the 96 well plate (see FIG. 8). The stage should securely fix the microplate so that 4 of the "H" reservoirs (FIG. 1), which span 25 mm, fit the optical window of the instrument. In a 96 well microplate format, the first 4 "optical" H1, 2, 3, and 4 reservoirs (FIG. 1) are positioned over the target area. The well plates are then shifted in increments of 4 "optical windows" so that alignment and imaging can accommodate all 12 of the H reservoirs in 3 steps. The three positions of interest are shown and labeled in the diagram as position #1, #2, or # 3 (FIG. 1).

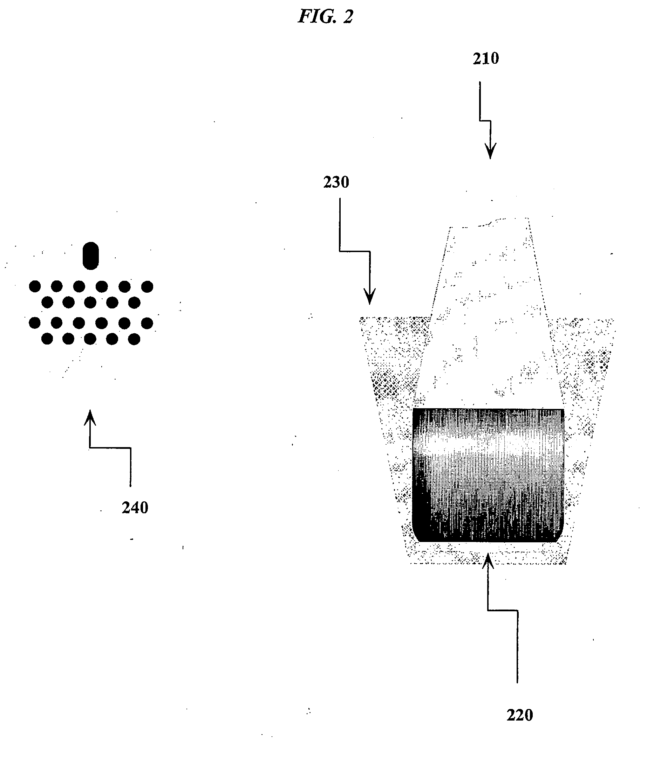

Arrangements involving plates pre-loaded with reagents and Footpads™ sealed with foil allow the operator to conveniently run from 1 to 12 test samples by loading only luminol-peroxide and sample into the microplate. Each row 1 through 12 represents a single sample process. With a breakaway row and seal the packaged kit can be used for incremental tests until all 12 rows are consumed, providing the stage adjusts to lock the "optical" well H on the target imager. Each Footpad™ in an arrayed format accommodates up to 24 spotted probes (FIG. 2) and allows for the possibility of multiplexing a single sample. FIG. 2 illustrates an exemplary arrangement, with an Eppendorf pipet tip 214 affixes to the top of a Footpad™ with a probe binding surface on the bottom 220, the Eppendorf tip surrounded by a reservoir with fluid 230. With a 96-swell format, the Footpad™ surface 220 is about 7 mm in diameter. An exemplary pattern of capture antibodies (200 µM spots) and index spot is also shown 240.

Using a "blank" non-arrayed Footpad™ in a 96 well microplate with an empty "antibody cocktail" reservoir allows the user to test for any targets of interest. This is accomplished by either manually spotting primary antibodies onto a pre-treated Footpad™, or affixing a robotically spotted cellophane, translucent nitrocellulose, rayon or other array of spots to the Footpad™. The operator then fills the empty reservoir with the biotinylated secondary antibodies, the H reservoir with luminol-peroxide, and the A reservoir with sample for testing (FIG. 1.).

Since 96 well plates are easily obtained, inexpensive, and optically suitable for a bioluminescent assay, and because preloading the plate is straight forward, one format for the bioluminescent TOAD^{™} may include a stage to accommodate the 96 well plate in the manner indicated.

A variety of samples may be analyzed using the methods and apparatus disclosed herein, including virtually any type of environmental, clinical, veterinary, pathologic or medical sample. In different arrangements, samples may be analyzed after collection without any sample treatment whatsoever. In other arrangements, samples may be processed after collection and before analysis. Samples may be processed in various ways, including without limitation cooling, freezing, heating, homogenization, organic phase extraction, detergent extraction, enzymatic digestion, centrifugation, filtration, ultracentrifugation, ultrafiltration, lyophilization or various well known chromatographic procedures. In certain arangements, it is anticipated that solid samples may be treated by homogenization and, if necessary, crude filtration, such as filtering the sample through a nylon or other filter with a pore size of approximately 100 µm or less.

### Exemplary Footpad™ and Sporting Pattern

In certain forms, TOAD™ Footpads™ are cylinders measuring 7 mm in height and 7 mm in diameter, fitting with the lower flat coated surface resting on the flat bottom of the well reservoir (FIG. 2). The exposed upper surface may be used to affix an inverted eppendorf pipet tip, handle or appendage so that the pad may be moved easily from one reservoir to the next. The lower surface of the Footpad™ is coated with an appropriate binding medium and may be either blank or pre-arrayed (spotted) with capture antibodies (probes). Each pad may be marked with an index calibration spot (FIG. 2) serving two purposes.

The index when placed in luminol-peroxide provides a reference for luminosity based on a standard spot. It also serves to identify any other spots that react in a test since the position relative to the index is always known. It does not matter if the pad rotates in the reservoir, since capture probe spots are identified in spatial relationship to the index calibration spot. All positions are correctly readable as long as the pad is placed face (treated surface) down in a reservoir.

A total of 24 spots may be affixed to a single Footpad™ without exceeding a density of 100 spots per square civil. A binary ID code may be affixed to a Footpad™, for example using the first two rows of spots, and used to provide a tracking lot ID number.

### Translucent nitrocellulose binding surface affixed to the Footpad™

The lower surface of the Footpad™ may be used to accept and firmly affix probes. The composition of the surface may be important for optimized probe binding and analyte detection and quantification. Depending upon how the array or spotting is done, the composition may vary. In preferred, arrangements, the lower surface of the Footpad™ is coated with a thin layer of translucent nitrocellulose.

Colloidal 1% nitrocellulose firmly affixes to nylon, and to a lesser extent to other plastics and glass. As shown herein, the translucent nitrocellulose surface formed after drying on these surfaces avidly binds proteins and other substrates, while preserving their functionality (e.g., enzyme activity, antibody-antigen binding, etc.) A Footpad™ of nylon to which Translucent nitrocellulose has been applied may be used. This format works well for manual spotting on the pad but may be less useful for robotic prayers, which are mostly designed to print on ordinary slides measuring 25 x 75 x 1 mm.

In some arrangements, a sheet of nylon coated with nitrocellulose may be used to print the array. The array may then be punched out and affixed to the lower surface of the Footpad™ with an adhesive. In alternative arrangements, a device may be constructed to hold coated Footpads™ firmly in position for robotic array work. Additional details of arrangements using translucent nitrocellulose layers for probe binding are disclosed below.

### Rayon and Cellophane Substrates

We have shown that 100% rayon thread, when immersed in a phosphate buffer with biotinylated BSA, affixed the B-BSA and produces a strong signal easily seen in the HRP-SA Luminol-peroxide format on the bioluminescent detector. The signal is intense and specific for biotin affixed to the thread, as can be demonstrated by first blocking the thread with 0.1% BSA. No signal is evident if the thread is blocked first before adding HRP-SA and Luminol-peroxide. It appears that rayon, a naturally occurring cellulose polymer, behaves in a manner similar to nitrocellulose. Untreated "raw" cellophane is identical to 100% rayon and can be obtained in translucent thin sheets. It is abundant and inexpensive.

In some arrangements, functional Footpads™ to bind probes may be manufactured out of rayon. Alternatively, cellophane printed discs may be affixed to the Footpad™ with an appropriate adhesive. The array may be printed on a thin sheet of cellophane before affixing it to glass, plastic, or any other solid support.

The Footpad™ may be of any color and does not have to be translucent. A rayon or cellophane surface requires no solvent or drying step, making the manufacturing process less complicated than the colloidal nitrocellulose process for coating either slides or Footpads™.

### 96 Well Microplate Procedure

In the following discussion, HRP-SA reagent refers to horse radish peroxidase conjugated with streptavidin in a solution formulated to enhance antigen detection. The solution is normally stable for at least 2 years from the time of formulation. Its activity can be verified in a test by placing one drop into 100 µL of luminol-peroxide solution. In a totally dark room, the faint blue light emitted shows that the light-producing reaction has begun.

The Footpads™ are either precoated and spotted with probes or precoated and blank (no spots on the pad), placed in the empty reservoir A column (FIG. 1). The operator using a blank (non-pre-arrayed) package will conduct assays with probes and biotinylated antibodies that are provided by the user. Because the Footpad™ comes already prepared for manual spotting, the operator needs only to remove the Footpad™, apply spots to its lower surface, fill the antibody D reservoir (FIG. 1) with biotinylated antibodies, fill the luminol-peroxide H reservoir (FIG. 1), block the Footpad™, and begin the test.

### Material List Using Pre-coated blank Footpad™ Test

The following materials may be used for a pre-coated blank Footpad™ test.
Pre-coated blank Footpad™ 96 well kit
Spotting buffer
Primary antibody for target of interest
Secondary biotinylated antibody to form the sandwich immunocomplex Antibody dilution buffer with stabilizer
Index marking solution
Luminol-peroxide reagent
Sample for testing
TOAD^{™} bioilluminator
Desktop computer and software
*Material List Pre-arrayed blank Footpad*™ *Test*

For pre-arrayed Footpad™ tests, the following materials may be used.
Pre-arrayed Footpad™ 96 well kit
Luminol-peroxide reagent
Sample for testing
TOAD^{™} bioilluminator
Desktop computer and software
*Pre-coated Blank Footpad*™ *Procedure*

All reagents and materials may be assembled and identified before initiating a test. In this embodiment, the user has elected to use antibodies for probes and secondary biotinylated antibodies to capture a target when using a blank Footpad™. The procedure is illustrated in Table 1.

### Pre-Arrayed Footpad™ Procedure

All reagents and materials may be assembled and identified before initiating a test. In this embodiment, the user has elected to use a pre-arrayed Footpad™ configuration, making the entire process considerably simpler and faster using spotted Footpads™. Only two solutions are loaded into well reservoirs. The luminol-peroxide reagent is mixed and added to reservoir H (FIG. 1). The sample is placed in reservoir A (FIG. 1). Processing begins after the Footpad™ has been adequately blocked, incubating 5 minutes in reservoir B. The procedure is illustrated in Table 2.

### Component Configuration

Exemplary kit components of potential use with a Footpad™ format are indicated in Table 3.

### Binary ID Code

A binary code of spots may be used as an index to track production batches of pre-arrayed Footpads™; GRABBER™ slides or other items. An exemplary method of coding is disclosed herein. A binary code system to identify a production particular lot may be used in the first two rows of the Footpad™ (FIG. 2). There are 3 reference spots in the first calibrator position. The reference spots are used to determine if the other spots in the adjacent rows and column are binary 1 or binary 0. A 4^{th} spot located at the coordinates for row 2 column 2 is a quality assurance check sum on the intended code for the lot number assigned. The other spots in the first two rows are either empty or filled with the biotin marker at the same concentration and volume as is used for calibration spots and are determined to be either 0 or 1 by comparison to the 3 binary reference marks.

### Binary 0 reference spot

The binary 0 reference spot contains 1/10^{th} of the concentration of the biotin marker used in a standard calibration spot. If any of the spots used to set the binary code are measured with a luminosity of lower intensity than the binary 0 reference, they are assigned a value of 0. A spot measured with a greater luminosity than both the binary 0 reference and the binary 1 reference is assigned the value of 1. A spot with a measured luminosity in between the binary 0 and binary 1 reference spots registers as a defective Footpad™ and is reported as an error to the operator.

### Binary 1 reference spot

The binary 1 reference spot contains 3/10^{th} of the concentration of the biotin marker used in a standard calibration spot. If any of the spots used to set the binary code are measured with a luminosity of greater intensity than the binary 1 reference, they are assigned a value of 1. A spot of lower luminosity than both the binary 1 reference and the binary 0 reference is assigned the value of 0. A spot measuring luminosity between the reference spots registers as a defective Footpad™ and is reported as an error to the operator.

### Binary Code and Lot Number Assignments

For each production of a Footpad™ there is a lot production date. The date of production may serve as the Lot number assignment for that production. The date may be printed in a binary code format. The unique pattern for a particular production date may be determined when a request for a lot production run is made.

A proprietary software program coding and assigning filled and empty spots may be used so that a user by entering the date for the production run will see the spots that are to be filled and those to be left empty. The program used for the pattern of filled spots in the ID code also generates the assignment for the check-sum validation reference.

### Check Sum Validation Spot

A reference spot at row 2 column 2 may be either completely filled or left empty as a validation of the binary code on the Footpad™. If the sum of the value 1's in the binary code assigned for a particular date is an even number, then the value 0 (empty spot) may be assigned for the check sum validation spot. If the sum of the value 1's in the binary code assigned for a particular date is an odd number, then the value 1 (filled spot) may be assigned for the check sum validation spot. The check sum validation when read against the binary code reference spots will be either 0 or 1. If the value read fails to match the assigned value for a particular production date, the Footpad™ will be assumed to have been printed incorrectly and discarded.

### GRABBER™ Slides

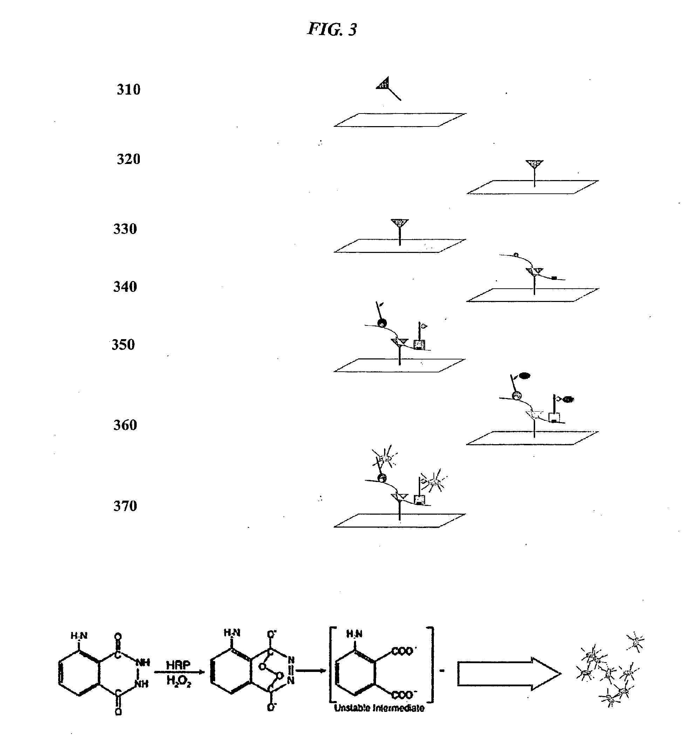

Arrangements concerning a microtiter well format are discussed above (Footpad™). Alternative arrangements concern devices and methods of use of translucent coated slides (GRABBER™) (FIG. 3). FIG. 3 illustrates an exemplary protocol for use with GRABBER™ slides, comprising affixing a primary antibodies to the slide 310, drying the 2D array 320, blocking the slide 330 (10 min), capturing antigen with the primary antibodies 340 (10 min), forming an immunocomplex with secondary biotinylated antibodies 350 (10 min), forming HRP-SA complexes 360 (10 min), adding luminol reagent 370 and capturing an image (10 min). The luminescent reaction catalyzed by HRP is as indicated in the bottom of FIG. 3.

GRABBER™ slides provide for an easy to use method for detecting and identifying one or more targets of interest in a solution assayed on the surface of a slide. Slides may be provided pre-spotted with capture probes (e.g., antibodies) affixed ready for immediate testing. Alternatively, users who want to prepare their own 2D-spotted array may in 1 hour prepare a manual array for testing.

In preferred arrangements, the method of detection utilizes a primary antibody capturing an antigen target to form an immunocomplex with a second antibody that is biotinylated or otherwise labeled. The first antibody probe is affixed to a translucent nitrocellulose slide (GRABBER™) and during the entire procedure remains affixed to the surface of the slide.

The immunocomplex, if formed, may be detected using a well-known enzyme activated bioluminescent process. Horseradish peroxidase (HRP) catalyzes the breakdown of peroxide and produces an intense light if luminol is added to the solution during the breakdown process. Luminol forms an unstable free radical intermediate that results in the release of photons at 430 nm (FIG. 3). By conjugating HRP to streptavidin (SA), the immunocomplex may be detected with an appropriate and sensitive photon detector. Biotin and streptavidin rapidly combine to form this light producing complex.

Typical protein (e.g., antibody) probes may be spotted with 1 to 5 nL per spot at a concentration of 0.05 to 0.2 mG/mL. Where primary antibodies are attached to a slide, matrix array or other surface, secondary tagged antibody, such as biotinylated antibody, may be provided in a dilution buffer premixed at an optimized concentration for target detection with pre-spotted slides. The optimized concentration may be determined empirically by the user. Secondary antibodies may be diluted in dilution buffer to a concentration typically in the range of 5 to 30 µG/mL.

A TOAD™ (total optical assay device) may be provided with proprietary software to allow the user the means to read, interpret and record signals at the surface of a slide. An image file and report may be saved or printed for subsequent analysis and comparisons.

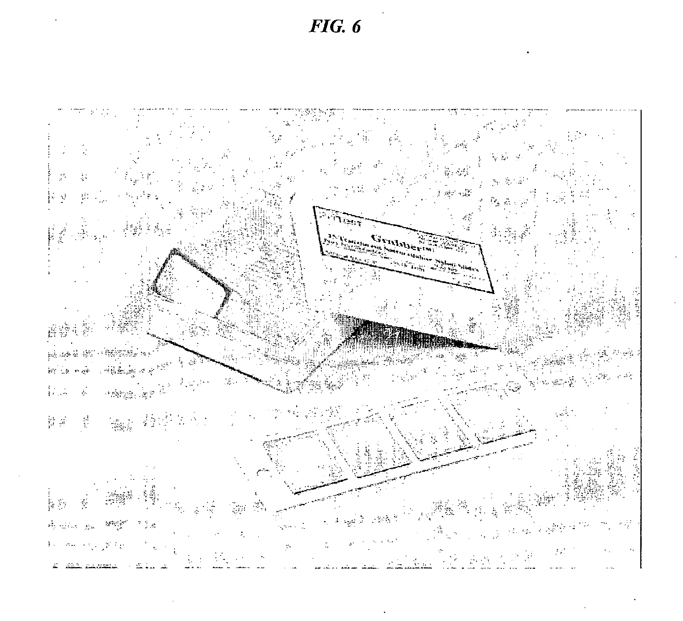

The user may provide a sample that is placed in an incubation sleeve (FIG. 6) affixed to the slide and allowed to react for 10 minutes before it is discarded (see below).

GRABBER™ slides may be coated with a translucent nitrocellulose substrate that avidly and immediately binds proteins, carbohydrates and/or oligonucleotides, strongly affixing them to the coaled surface while preserving the functionality and 3D structure of the bound molecule. No other chemical process is required to affix the probes to the slide surface, making the 2D array procedure simple and fast. The hydrophobic surface with a superior contact angle is well suited for compact robotically printed arrays.

The bioluminescent reaction is long lasting and the signal may be acquired over prolonged periods, allowing for extraordinary sensitivity in detection. Many targets may be simultaneously identified in a single sample, The proper primary probe antibody and biotinylated secondary antibody should strongly interact with the target for adequate detection. Preprinted slides and reagents provided are optimized to produce high quality detection.

### Materials List

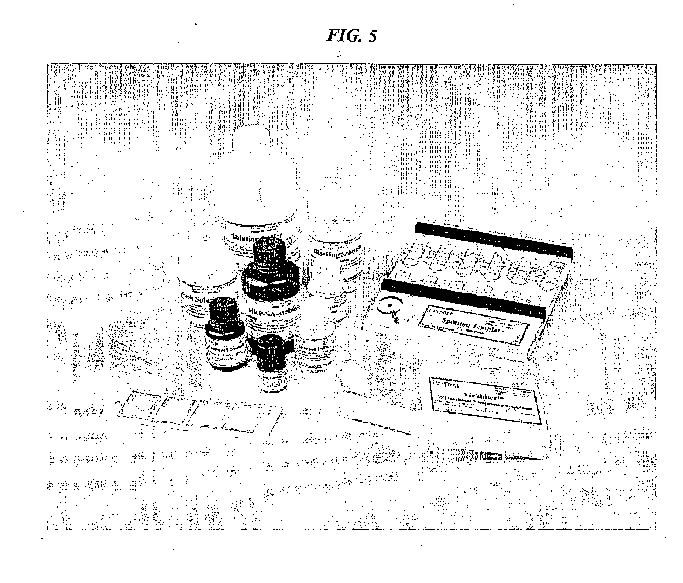

All of the disposable slides, reagents and other materials needed to assay for analytes using GRABBER™ slides with a TOAD™ bioilluminator may be provided in kit form, as illustrated in FIG. 5. FIG. 6 shows an exemplary set of GRABBER™ slides, along with an incubation sleeve for performing the binding, washing and other procedures on the slide. The exemplary incubation sleeve shown in FIG. 6 contains four separate reaction chambers for use with GRABBER™ slides.

The following exemplary materials may be used to perform a pre-coated nitrocellulose slide assay. Non-limiting exemplary kit materials for use with GRABBER™ slides are also listed in Table 7, along with exemplary sources for the materials. A kit may be provided with all the materials needed to complete an assay. The user may assemble gloves, forceps, a timer, waste container for spent material, and eppendorf tips for spotting an array. A sample for testing should be acquired before beginning the assay. If the array is pre-spotted, testing may begin with the blocking as shown in FIG. 3.
GRABBER^{™} pre-coated slide
Spotting buffer
Spotting template
Focus Template slide
Primary antibody for target of interest
Secondary biotinylated antibody to form the sandwich immunocomplex
Antibody dilution buffer with stabilizer
Index marking solution
HRP-SA reagent
Luminol-peroxide reagent
Sample for testing
Incubation sleeve
TOAD™ bioilluminator
Desktop computer and software
Disposable dispensers
Timer
Dust free latex gloves
Forceps

### Pre-spotted Array Protocol

All reagents and materials may be assembled and identified before initiating an assay. In certain embodiments, pre-spotted arrays ready for testing are obtained. Slides have incubation sleeves (FIG. 6) affixed to the nitrocellulose surface and secondary biotinylated antibodies are provided ready for use. An exemplary protocol for use with pre-spotted arrays is disclosed in Table 4.

### Protocol for User Prepared 2D Array

In an alternative embodiment, the user may elect to provide primary antibodies for probes and secondary biotinylated antibodies to detect a target when using a pre-coated non-arrayed slide. An exemplary protocol is shown in Table 5.

### Detection Unit

The discussion above illustrates the use of a TOAD™ containing an exemplary CCD based optical detection system. In certain arrangements, a TOAD™ may comprise one or more alternative detection units. Optical signals may be produced when analytes bind to probes and HRP-conjugated secondary antibodies in the presence of luminol, as disclosed above. The signals may be detected by the detection unit. The detection unit may comprise one or more detectors, such as a spectrometer, monochromator, CCD device, CCD camera, photomultiplier tube, photodiode, avalanche photodiode or any other device known in the art that can detect an optical signal. An optical signal may comprise any form of electromagnetic radiation, emission, or absorption, although in preferred embodiments the optical signal comprises visible light.

In preferred arrangements, the presence of analyte attached to one or more probes is indicated by a bioluminescent spot on the surface of the slide, 96-well microplate or other surface. In various arrangements, the device may comprise a data analysis and storage unit, such as a computer or microprocessor. Suitable devices are well known in the art and the present invention is not limiting as to the type of data analysis and storage unit used. The data analysis and storage unit may be operably coupled to the detection unit, so that optical signals are collected, processed and stored. In alternative embodiments, the data analysis and storage unit may store information on each sample collected, including the sample source, geographical location and any other data collected on the sample. The unit may further identify each analyte detected in the sample and store that data as well. In certain arrangements, the device may comprise an interface for downloading data to a remote location, either directly (for example by an incorporated radio transmitter or modem) or indirectly (for example, by downloading to diskette or other storage device or by printing a hard copy).

In certain arrangements, one or more optical components may be interposed between the spot containing surface and detector, such as a lens or lens array to focus optical signals from each spot. A non-limiting example of a detector may comprises a CMOS camera sensor or equivalent unit, such as a PixeLink model AL633 CMOS imager (Vitana Corp., Ottawa, Canada).

In an exemplary arrangement, a nylon slide or other matrix Stray may be secured on a stage. A CMOS imager may be used to capture the light signals. The CMOS chip may be located beneath the slide and aligned so that spots on the slide are directly above the imager and are sharply focused on the imager surface with optical lenses and apertures.

### Probes

Various embodiments concern the use of probes for the detection of analytes. Although in preferred embodiments the probes are antibodies, it is contemplated that virtually any molecule or aggregate that can bind to a target analyte with sufficient affinity and specificity to allow its detection may be used. Standard procedures for the production of monoclonal or Polyclonal antibodies are known (see, *e.g.*, Harlow and Lane, Antibodies: A Laboratory Manual, Cold Spring Harbor Laboratory, Cold Spring Harbor, New York, 1988; incorporated herein by reference).

Polyclonal antibodies are prepared by immunizing an animal with an immunogen and collecting antisera from the immunized animal. A wide range of animal species can be used for the production of antisera. Typical animals used for production of anti-antisera include, for example, rabbits, mice, rats, hamsters, pigs or horses. Because of the relatively large blood volume of rabbits, a rabbit is a preferred choice for production of polyclonal antibodies.

Antibodies, both polyclonal and monoclonal, may be prepared using conventional immunization techniques, as will be generally known to those of skill in the art. A composition containing antigenic epitopes of an analyte can be used to immunize one or more experimental animals, such as a rabbit or mouse, which will then proceed to produce specific antibodies against the analyte. Polyclonal antisera may be obtained, after allowing time for antibody generation, simply by bleeding the animal and preparing serum samples from the whole blood.

A given composition may vary in its immunogenicity. It is often necessary, therefore, to boost the host immune system, as may be achieved by coupling an immunogen to a carrier. Exemplary and preferred carriers are keyhole limpet hemocyanin (KLH) and bovine serum albumin (BSA). Other albumins such as ovalbumin, mouse serum albumin or rabbit serum albumin also can be used as carriers. Means for conjugating an analyte to a carrier protein are well known in the art and include glutaraldehyde, *m*-maleimidobenzoyl-N-hydroxysuccinimide ester, carbodiimide and bis-biazotized benzidine.

The immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed *Mycobacterium tuberculosis),* incomplete Freund's adjuvants and aluminum hydroxide adjuvant.

The amount of immunogen composition used in the production of polyclonal antibodies varies, depending upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster, injection also may be given. The process of boosting and titering is repeated until a suitable titer is achieved. When a desired level of immunogenicity is obtained, the immunized animal can be bled and the serum isolated and stored, and/or the animal can be used to generate monoclonal antibodies.

Monoclonal antibodies may be readily prepared through use of well-known techniques, such as those exemplified in U.S. Patent 4,196,265, incorporated herein by reference. Typically, this technique involves immunizing a suitable animal with a selected immunogen composition, *e.g*., a purified or partially purified composition comprising an analyte. The immunizing composition is administered in a manner effective to stimulate antibody producing cells. Cells from rodents such as mice and rats are preferred, however, the use of rabbit, sheep or frog cells is also possible.

Following immunization, somatic cells with the potential for producing antibodies, specifically B-lymphocytes (B-cells), are selected for use in the mAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of the animal with the highest antibody titer will be removed and the spleen lymphocytes obtained by homogenizing the spleen with a syringe. Typically, a spleen from an immunized mouse contains approximately 5 × 10⁷ to 2 × 10⁸ lymphocytes.

The antibody-producing B lymphocytes from the immunized animal are then fussed with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas).

Any one of a number of myeloma cells may be used, as are known to those of skill in the art. For example, where the immunized animal is a mouse, one may use P3-X63/Ag8, P3-X63-Ag8.653, NS1/1.Ag 4 1, Sp210-Agl4, FO, NSO/U, MPC-11, MPC11-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with cell fusions.

Methods for generating hybrids of antibody-producing spleen or lymph node cells and myeloma cells usually comprise mixing somatic cells with myeloma cells in a 2:1 ratio, though the ratio may vary from about 20:1 to about 1:1, respectively, in the presence of an agent or agents (chemical or electrical) that promote the fusion of cell membranes. Fusion methods using Sendai virus (Kohler and Milstein, Nature, 256:495-497, 1975; Eur. J. Immunol., 6: 511-519, 1976), and those using polyethylene glycol (PEG), such as 37% (v/v) PEG, have been disclosed (Gefter et al., Somatic Cell Genet., 3: 231-236, 1977). The use of electrically induced fusion methods is also appropriate (Goding, In: Monoclonal Antibodies: Principles and Practice, 2d ed., Academic Press, Orlando, Fla., pp. 60-61, and 71-74, 1986).

Fusion procedures usually produce viable hybrids at low frequencies, around 1 × 10⁻⁶ to 1 × 10⁻⁸. However, the viable, fused hybrids may be differentiated from the parental, unfused cells by culturing in a selective medium that contains an agent that inhibits cell growth for unfused cells. Exemplary agents are aminopterin, methotrexate, and azaserine. Where aminopterin or methotrexate are used, the medium is supplemented with hypoxanthine and thymidine as a source of nucleotides (HAT medium). Where azaserine is used, the medium is supplemented with hypoxanthine.

A preferred selection medium is HAT. Only cells capable of operating nucleotide salvage pathways are able to survive in HAT medium. The myeloma cells are defective in key enzymes of the salvage pathway, *e.g.,* hypoxanthine phosphoribosyl transferase (HPRT), and they cannot survive. The B-cells can operate this pathway, but they have a limited life span in culture and generally die within about two weeks. Therefore, the only cells that can survive in the selective media are those hybrids formed from myeloma and B-cells.

This culturing provides a population of hybridomas from which specific hybridomas are selected. Typically, selection of hybridomas is performed by culturing the cells by single-clone dilution in microtiter plates, followed by testing the individual clonal supernatants (after about two to three wk) for the desired reactivity. The assay should be sensitive, simple and rapid, such as radioimmunoassays, enzyme immunoassays, cytotoxicity assays, plaque assays, dot immunobinding assays, and the like.

The selected hybridomas would then be serially diluted and cloned into individual antibody-producing cell lines, which clones can then be propagated indefinitely to provide mAbs. The cell lines may be exploited for mAb production in two basic ways. A sample of the hybridoma can be injected (often into the peritoneal cavity) into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can then be tapped to provide mAbs in high concentration. The individual cell lines also could be cultured *in vitro,* where the mAbs are naturally secreted into the culture medium from which they can be readily obtained in high concentrations. Monoclonal antibodies produced by either means may be further purified, if desired, using filtration, centrifugation, and various chromatographic methods such as HPLC or affinity chromatography.

Although the methods and compositions disclosed herein allow for the production of novel antibodies against analytes, it is contemplated that previously characterized antibodies or commercially available antibodies may be used to bind to and detect any analyte of interest.

Non-antibody probes that may be used include, for example, aptamers (*e.g*., U.S. Pat. Nos. 5,270,163; 5,567,588; 5,670,637; 5,696,249; and 5,843,653, peptide libraries (*e.g*., U.S. Patent Nos. 5,565,332, 5,596,079, 6,031,071 and 6,068,829, and various receptor proteins, binding proteins, cell surface proteins, and other non-antibody peptides or proteins known in the art. **Probe Labels**

Labeled probes may be prepared by any methods known in the art. In certain arrangements, a label moiety may be incorporated into a probe (*e.g*., peptide, protein, oligonucleotide) during synthesis. In other arrangements labels may be attached by covalent, noncovalent, ionic, van der Waals, hydrogen bonding or other forces following probe synthesis. Methods for attaching fluorescent or other labels to probe molecules are known in the art and any such known method may be used to make labeled probes within the scope of the present invention, In particular arrangements, a probe molecule may be biotinylated and may bind to an avidin or streptavidin-conjugated fluorophore. Fluorophores and conjugated fluorophores may be obtained from commercial sources, such as Molecular Probes, Inc. (Eugene, OR).

Labels of use may comprise any composition detectable by electrical, Optical, spectrophotometric, photochemical, biochemical, immunochemical, or chemical techniques. Labels may include, but are not limited to, conducting, luminescent, fluorescent, chemiluminescent, bioluminescent and phosphorescent labels, chromogens, enzymes or substrates. Fluorescent molecules suitable for use as labels include, but are not limited to, dansyl chloride, rhodamineisothiocyanate, Alexa 350, Alexa 430, AMCA, BODIPY 6301650, BODIPY 650/665, BODIPY-FL, BODIPY-R6G. BODIPY-TMR, BODIPY-TRX, Cascade Blue, Cy2, Cy3, Cy5,6-FAM, fluorescein, HEX, 6-JOE, Oregon Green 488, Oregon Green 500, Oregon Green 514, Pacific Blue, REG, Rhodamine Green, Rhodamine Red, ROX, TAMRA, TET, Tetramethylrhodamine, and Texas Red A variety of other known fluorescent or luminescent labels may be utilized. (*See, e.g.,* U.S. Pat. No. 5,800,992; U.S. Par. No. 6,319,668.)

### General Matters

In the description above, for the purposes of explanation, numerous specific details are set forth in order to provide a thorough understanding of the present invention. It will be apparent, however, to one skilled in the art that the present invention may be practiced without some of these specific details. In other instances, well-known structures and devices are shown in block diagram form.

The present invention may include various processes. The processes of the present invention may be performed by hardware components or may be embodied in machine-executable instructions, which may be used to cause a general-purpose or special-purpose processor or logic circuits programmed with the instructions to perform the processes. Alternatively, the processes may be performed by a combination of hardware and software.

Many of the methods are described in their most basic form, but processes can be added to or deleted from any of the methods and information can be added or subtracted from any of the described messages without departing from the basic scope of the present invention. It will be apparent to those skilled in the art that many further modifications and adaptations can be made. The particular embodiments are not provided to limit the invention but to illustrate it. The scope of the present invention is not to be determined by the specific examples provided above but only by the claims below.

It should also be appreciated that reference throughout this specification to "one embodiment" or "an embodiment" means that a particular feature may be included in the practice of the invention. Similarly, it should be appreciated that in the foregoing description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims are hereby expressly incorporated into this description, with each claim standing on its own as a separate embodiment of this invention.

### EXAMPLES

### Example 1. Microarray Formation Using Translucent Nitrocellulose Coated Slides

Colloidal nitrocellulose slides may be spotted with probes using any known methods for microarray production. Methods for spotting protein, peptides, oligonucleotides and nucleic acids onto nitrocellulose surfaces are well known in the art. Antibodies and biotinylated bovine serum albumin were used to determine the colloidal nitrocellulose binding capacity. The estimated protein binding capacity for the initial matrix arrays was in the range of 100 to 200 µg/cm².

A CMOS imaging system that detected light emissions through the translucent nitrocellulose matrix arrays was used with a Cy5-streptavidin indicator dye. (See, *e.g*., US 6767733) At a loading volume of approximately 5 nanoliters per spot, biotinylated BSA was reproducibly detected at least down to 10 to 20 picograms of protein. Optimal CMOS images were obtained using a protein concentration range of about 20 to 100 µg/mL biotinylated BSA. The CMOS imaging data was confirmed using a 24 hour colloidal gold stain (BioRad, Hercules, CA) of the spot arrayed translucent nitrocellulose slides.

The ability of the colloidal nitrocellulose matrix to maintain antibody binding activity was confirmed by spotting a variety of antibodies on the translucent surface. A concentration range of 20 to 200 µg/mL was used. Nonspecific protein binding sites on the nitrocellulose membrane were blocked with 0.1% BSA in buffer solution. The antibodies were then exposed to solutions containing the appropriate target antigen. Binding was detected using biotinylated second antibodies and Cy5-streptavidin indicator.

A 2D array spotted with 5 nanoliters per spot of primary goat anti-mouse antibody solution and was developed using a secondary mouse *Listeria* monoclonal antibody and a secondary biotinylated goat anti-mouse antibody. In this assay, the primary and secondary goat anti-mouse antibodies bound to different epitopes of the secondary mouse antibody. The two secondary antibodies were thus used to detect primary antibody bound to the array. Using a CMOS imaging system, the primary antibody could be detected down to a level of 100 picograms or less of antibody. The CMOS data was confirmed on the same slides using colloidal gold staining.

### Example 2. Bioluminescent Detection Sensitivity for Virus Analytes

Rous sarcoma virus (RSV) was titered using primary antibody probe binding and HRP-SA bioluminescent detection as disclosed above. Prespotted slides containing anti-RSV antibodies were prepared for RSV. RSV was serially diluted 10 fold with each dilution to finally reach a lower limit of detection where no further spots were detected. A lower limit of detection was estimated at is 0.000091 IU/mL for bioluminescent detection (10¹³ fold dilution from the initial stock titer).

All of the METHODS and APPARATUS disclosed and claimed herein can be made and executed without undue experimentation in light of the present disclosure. While the methods of this invention have been described in terms of preferred embodiments, it will be apparent to those of skill in the art that variations may be applied to the METHODS and APPARATUS and in the steps or in the sequence of steps of the methods described herein without departing from the scope of the invention. More specifically, it will be apparent that certain agents that are both chemically and physiologically related may be substituted for the agents described herein while the same or similar results would be achieved. All such similar substitutes and modifications apparent to those skilled in the art are deemed to be within the scope of the invention as defined by the appended claims.

**Table 1: Pre-coated Blank Footpad Procedure**

| | **Time (mins)** |
|---|---|
| Remove foil seal exposing first the footpad in the A reservoir. Carefully remove the footpad with an inverted eppendorf. | |
| Apply with an eppendorf a microspot index mark at the 12 o'clock position using an index marker. | 1 |
| Apply from 1 to approximately 8 additional microspots on the footpad in a pattern noting and recording accurately the position of each spot relative to the index. | 2 |
| Allow approximately 30 minutes air drying in a clean protected area. | 3 |
| Prepare secondary biotinylated antibodies diluting with dilution buffers to an appropriate level (approximately 10 - 30 uG/mL). Do not use sodium azide or other antimicrobial growth inhibitors because they will interfere in the test. Dilution buffers are formulated with the appropriate inhibitors.¹ | |
| Prepare the test sample that will be added to reservoir A. | |
| Prepare luminol-peroxide solutions mixing equal volumes of PriTest provided substrate. | 1 |
| Remove the foil covering reservoirs B through H. | |
| Add 100 uL luminol-peroxide substrate (step 8) to reservoir 8. Add 200 uL biotinylated antibody (step 6) to reservoir D. | |
| Place footpad face down into reservoir B (blocking solution) and allow 5 minutes incubation. | 5 |
| Place test sample (step 7) in reservoir A. | |
| Place footpad face down into reservoir A, and allow 10 minutes incubation. | 10 |
| Remove footpad from A reservoir, immerse briefly in B to wash the surface. Then move directly to reservoir C and briefly immerse and wash the surface, then place the footpad in D to incubate 10 minutes. | 10 |
| Remove the footpad from D reservoir, wash in E briefly, and place it in reservoir F to incubate 10 minutes. ¹ProClin 0.05%. | 10 |
| Remove the footpad from F reservoir, wash in G briefly, and place it in reservoir H. | |
| Immediately place the microplate on the TOAD stage and begin imaging. | |
| Imaging may take 15 minutes for very low level detection. | 15 |

**Table 2: Pre-Arrayed Footpad Procedure**

| | **Time (mins)** |
|---|---|
| Remove foil seal exposing first the footpad in the A reservoir. | |
| Carefully remove the footpad with an inverted eppendorf from reservoir A and place it face down in reservoir B (blocking solution) and allow 5 minutes incubation Prepare the test sample that will be added to reservoir A. | 5 |
| Prepare luminol-peroxide solutions mixing equal volumes of PriTest provided substrate. | 1 |
| Remove the foil covering reservoirs C through H. | |
| Add 100 uL luminol-peroxide substrate (step 4) to reservoir 8. | |
| Place test sample (step 3) in reservoir A. | |
| Remove the footpad from reservoir B and place the footpad face down into reservoir Allow 10 minutes incubation. | 10 |
| Remove footpad from A reservoir, immerse briefly in B to wash the surface. Then move directly to reservoir C and briefly immerse and wash the surface, then place the footpad in D to incubate 10 minutes. | 10 |
| Remove the footpad from D reservoir, wash in E briefly, and place it in reservoir F to incubate 10 minutes. | 10 |
| Remove the footpad from F reservoir, wash in G briefly, and place it in reservoir H. | |
| Immediately place the microplate on the TOAD stage and begin imaging. | |
| Imaging may take 15 minutes for very low level detection. | 15 |

**Table 3: Component Configuration:**

| **Item** | **Composition** | **Units per 96 Well Microplate (per well/total)** | **Well** |
|---|---|---|---|
| Footpad | E.g. rayon, nylon, cellophane | 1/12 | A |
| Spotting buffer | PBS (phosphate buffered saline) | N/A | |
| Dilution buffer | 0.1% BSA, PBS | N/A | |
| | 0.05% ProClin 950 | | |
| Index buffer | PBS, 0.05% ProClin 950 | N/A | |
| | 30 uG/mL B-BSA | | |
| Locking solution | PBS, 0.1% BSA | 100 uL/1200 uL | B |
| | 0.05% ProClin 950 | | |
| Wash solution | PBS | 100 uL/ 3600 uL | C |
| | 0.1% BSA | | E |
| | 0.05% ProClin 950 | | G |
| | 0.03% Triton X100 | | |
| HRP-SA | PBS, 0.1% BSA | 100 uL/1200 uL | |
| | 0.05% ProClin 950 | | F |
| | 0.03% Triton X100 | | |
| | 0.1 uG/mL HRP-SA | | |
| | | | |
| Luminol-peroxide | Mix 1:1 volumes prior to | | |
| | test procedure and fill H | 100 uL/1200 uL | H |
| | reservoir | | |
| | | | |
| Antibody Cocktail | PBS | | |
| | 0.1% BSA | | |
| | 0.05% ProClin 950 | 100 uL/1200 uL | D |
| | 0.03% Triton X100 | | |
| | Biotinylated Antibodies | | |

**Table 4: Pre-spotted Array Procedure**

| | **Time (min)** |
|---|---|
| Prepare the test sample that will be used. The sample may be immobilized in dilution buffer. | |
| | |
| Prepare luminol-peroxide solutions mixing equal volumes of provided substrate. | 1 |
| Dilute the mixed reagents with a volume of dilution buffer equal to the total luminol + peroxide (example: 1 mL Luminol + 1 mL peroxide, then dilute with 2 mL dilution buffer to produce 4 mL total test reagent). | |
| | |
| Block the prepared slide by filling the incubation sleeve chamber with blocking solution and allowing 10 minutes for complete blocking. Then remove and dispose of blocking solution. | 10 |
| | |
| Place test sample into the incubation chamber and allow 10 minutes for probe binding to occur. Then empty the reservoir contents and dispose of remaining sample. | 10 |
| | |
| Wash x 1 the incubation chamber with wash solution by completely filling and then emptying the reservoir chamber. | 1 |
| | |
| Fill the reservoir chamber with the secondary biotinylated antibody, and incubate 10 minutes. Then remove and dispose of the fluid. | 10 |
| | |
| Wash x 1 the incubation chamber with wash solution by completely filling and then emptying the reservoir chamber. | 1 |
| | |
| Fill the chamber with HRP-SA reagent and incubate 10 minutes. Then empty and dispose the spent reagent. | 10 |
| | |
| Wash twice (x 2) the incubation chamber with wash solution by completely filling and then emptying the reservoir chamber. | 2 |
| | |
| Fill the reservoir chamber with Luminol-peroxide reagent. Peel away the outer protective surface on the incubation sleeve, exposing the optically clear window. | |
| Place the slide on the TOAD stage. Position the slide to accurately view the target area. This may be confirmed using the Grid-template slide and centering the diamond as needed. | 1 |
| | |
| Open the software Slider2, and set the exposure time at 10 minutes | |
| | |
| Set the Readout speed at 2.5 Mhz. | 2 |
| | |
| Click "Capture Image". | 1 |
| | |
| After the image is obtained, make notes and save the file. The ID code on the slide may be used as the file name. | 1 |

**Table 5: Pre-coated Blank Slide Procedure**

| | **Time (min)** |
|---|---|
| With gloves, place a slide on the spotting template with the logo face up. The target area for probe spotting is between the diamond points. Mark a slide with a label using a black marker pen. | |
| | |
| Apply with an eppendorf pipette tip, a microspot index mark at one end of the target area spacing spots in a column. Note the position in a notebook, making a sketch of the slide. | 1 |
| | |
| Apply from 1 to 8 additional columns of microspots on the slide in a pattern, noting and recording accurately the position of each probe spot relative to the index in a notebook. Space columns at least 1 mm apart | 2 |
| | |
| Allow approximately 30 minutes air drying in a clean protected area. Prepare secondary biotinylated antibodies diluting with dilution buffers to an appropriate level (approximately 10 - 30 uG/mL). Do not use sodium azide or other antimicrobial growth inhibitors because they will interfere in the test. Dilution buffers are formulated with the appropriate microbial inhibitors.¹ | 30 |
| | |
| Affix the incubation sleeve to the slide, encompassing the spots deposited and air-dried. | 1 |
| | |
| Prepare the test sample that will be used. The sample may be immobilized in dilution buffer. | |
| | |
| Prepare luminol-peroxide solutions, mixing equal volumes of provided substrate. Dilute the mixed reagents with a volume of dilution buffer equal to the total luminol + peroxide (example: 1 ml Luminol + 1 mL peroxide, dilute with 2 mL dilution buffer to produce 4 mL total test reagent). | 1 |
| | |
| Block the prepared slide by filling the incubation sleeve chamber with blocking solution and allowing 10 minutes for complete blocking. Then remove and dispose of in a waste container. ¹ProClin 0.05%. | 10 |
| Place test sample into the incubation chamber and allow 10 minutes for probe binding. Then' empty the reservoir contents and dispose of in a waste container. | 10 |
| | |
| Wash x 1 the incubation chamber with wash solution by completely filling and then emptying the reservoir chamber. | |
| | |
| Fill the reservoir chamber with the secondary biotinylated antibody, and incubate 10 minutes. Then remove the fluid and dispose of in a waste container. | 10 |
| | |
| Wash x 1 the incubation chamber with wash solution by completely filling and then emptying the reservoir chamber. | 1 |
| | |
| Fill the chamber with HRP-SA reagent and incubate 10 minutes. Then empty and dispose of the spent reagent in a waste container. | 10 |
| | |
| Wash twice (x 2) the incubation chamber with wash solution by completely filling and then emptying the reservoir chamber. | 2 |
| | |
| Fill the reservoir chamber with Luminol-peroxide reagent. Peel away the outer protective surface on the incubation sleeve exposing the optically clear window and then place the slide on the TOAD stage. Position the slide to accurately view the target area. This may be confirmed using the Grid-template slide centering the diamond as needed. | 1 |
| | |
| Open the software Slider2, and set the exposure time at 10 minutes | 2 |
| | |
| Set the Readout speed at 2.5 Mhz. | |
| | |
| Click "Capture Image" | 1 |
| | |
| After the image is obtained, make notes and save the file. The ID code on the slide may be used as the file name. | |

**Table 6**

| **Part #** | **Description** | **Components** | **Supplier** | **PriTest Part #** | **Ref #** | **Quantity** |
|---|---|---|---|---|---|---|
| ***Instrument:*** | **Toad™ Illuminator** | | | FD-TI0001-0-001 | | |
| | **Toad™ Illuminator** | CCD | Qimaging | | QIC-M-10-C | 1 |
| | | PCI firewire card | Qimaging | | | 1 |
| | | Lens | Sunex | | DSL914D2 | 1 |
| | | Lens C-mount adapter | Paul Harris | FD | | 1 |
| | | Firewire 6 pin | Qimaging | | | 1 |
| | | Emerge Enclosure | Emerge | | | 1 |
| | | Software interface | Validio | | | 1 |
| | | Cooper Cooler-Heat | | | | |
| | | Sink: GF4Ti Cooper | Thermaltak | | | |
| | | Cooler | e | | A1349 | 1 |
| | | | | | | |
| | | Philmore DC Power | Fry's | | | |
| | | Jack 2.1mmx5.5mm | Electronics | | | 1 |
| | | CA-979/500mA AC | | | | |
| | | Universal PWR adaptor | Fry's | | | |
| | | UL | Electronics | | | 1 |
| | | Shipping carton | | | | 1 |
| | | Label | | | | 1 |

**Table 7**

| | **Grid Focus Slide** | | **PriTest** | FD-FS000G-0-001 | | 1 |
|---|---|---|---|---|---|---|
| | **Spotting Template** | | **PriTest** | FD-ST0005-0-001 | | 1 |
| **Part #** | **Description** | **Components** | **Supplier** | | **Ref #** | **Quantity** |
| ***Disposables:*** | | | | | | |
| | Grabber™ | Nylon 75mmx25mmx1nEmerge | | | | 1 |
| | | Nitrocellulose | | | | |
| | | Configuration 1 | | FD-GR0003-0-001 | | 25 |
| | | Configuration 2 | | | | 100 |
| | | | | | | |
| | Incubation sleeves | 500 uL adherent chambe | Gasket Sp | | Chamber BIC | 1 |
| | | Configuration 1 | | FD-TS0004-0-003 | Chamber BIC | 25 |
| | | Configuration 2 | | | Chamber BIC | 100 |
| | | | | | | |
| | Blocking Solution (mL) | BSA 0.1% w/v | Sigma | | A3803? | |
| | | ProClin 950 0.05% w/v | Supelco | | 46878-U | |
| | | Sigma PBS pack | Sigma | | P3813 | |
| | | Configuration 1 | | SK-BS1001-0-001 | | 100 |
| | | Configuration 2 | | | | 1000 |
| | | | | | | |
| | Dilution Buffer (mL) | BSA 0.1% w/v | Sigma | | A3803 | |
| | | ProClin 950 0.05% w/v | Supelco | | 46878-U | |
| | | Sigma PBS pack | Sigma | | P3813 | |
| | | Configuration 1 | | SK-DB1002-0-001 | | 500 |
| | | Configuration 2 | | | | 1000 |
| | | | | | | |
| | Wash Solution (mL) | BSA 0.1% w/v | Sigma | | A3803 | |
| | | ProClin 950 0.05% w/v | Supelco | | 46878-U | |
| | | Sigma PBS pack | Sigma | | P3813 | |
| | | Configuration 1 | | SK-WS1003-0-001 | | 100 |
| | | Configuration 2 | | | | 500 |
| | | | | | | |
| | Spotting Buffer (mL) | Sigma PBS pack | Sigma | | | |
| | | Configuration 1 | | SK-SB1004-0-001 | | 25 |

## Claims

1. A method of making translucent matrix arrays **characterised by**
a) in a first step coating a substrate with a translucent , optically clear, layer of colloidal nitrocellulose, wherein the colloidal nitrocellulose is suspended in either amyl acetate or other volatile organic solvents before application to the substrate which is in the form of a glass or other translucent slide,
and
b) in a second step attaching one or more first probes to the translucent, optically clear, colloidal nitrocellulose layer, wherein the translucent optically clear nitrocellulose layer has a protein binding capacity of 100-200 µg/cm².

2. The method of claim 1, wherein the substrate is glass or nylon.

3. The method of claim 1, wherein the first probes are selected from the group consisting of antibodies, antibody fragments, F Ab fragments, humanized antibodies, single-chain antibodies, chimeric antibodies, oligonucleotides, polynucleotides, nucleic acids, aptamers and affibodies.

4. The method of claim 1, wherein the matrix array is reconfigurable.

5. The method of claim 4, wherein the first probes are aptamers or affibodies.

6. The method of claim 5, wherein the first probes bind to 1gG (immunoglobulin G).

7. An optical assay device obtainable by the method of any one of claims 1 to 6, comprising:
a translucent, nylon slide;
a translucent, optically clear, colloidal nitrocellulose layer attached to the upper surface of the slide; and
a multiplicity of probes bound to the upper surface of the translucent, optically clear, colloidal nitrocellulose layer, wherein the translucent, optically clear, colloidal nitrocellulose layer has a binding capacity of 100-200 µg/cm².

## Patentansprüche

1. Verfahren zur Herstellung lichtdurchlässiger Matrixanordnungen, **gekennzeichnet durch**
a) Beschichten, in einem ersten Schritt, eines Substrats mit einer lichtdurchlässigen, optisch klaren Lage aus kolloidaler Nitrocellulose, wobei die kolloidale Nitrocellulose in Amylacetat oder anderen flüchtigen organischen Lösungsmitteln suspendiert wird, bevor sie auf das Substrat aufgetragen wird, das in Form eines Objektträgers aus Glas oder einem anderen lichtdurchlässigen Material vorliegt, und
b) Anfügen, in einem zweiten Schritt, von einer oder mehreren ersten Sonden an die lichtdurchlässige, optisch klare kolloidale Nitrocelluloselage, wobei die lichtdurchlässige, optisch klare Nitrocelluloselage eine Proteinbindekapazität von 100-200 µg/cm² hat.

2. Verfahren nach Anspruch 1, wobei das Substrat Glas oder Nylon ist.

3. Verfahren nach Anspruch 1, wobei die ersten Sonden ausgewählt sind aus der Gruppe bestehend aus Antikörpern, Antikörperfragmenten, Fab-Fragmenten, humanisierten Antikörpern, einkettigen Antikörpern, chimären Antikörpern, Oligonucleotiden, Polynucleotiden, Nucleinsäuren, Aptameren und Affibodys.

4. Verfahren nach Anspruch 1, wobei die Matrixanordnung umkonfigurierbar ist.

5. Verfahren nach Anspruch 4, wobei die ersten Sonden Aptamere oder Affibodys sind.

6. Verfahren nach Anspruch 5, wobei sich die ersten Sonden an IgG (Immunglobulin G) binden.

7. Optische Prüfvorrichtung, die mit dem Verfahren nach einem der Ansprüche 1 bis 6 erhältlich ist und Folgendes umfasst:
einen lichtdurchlässigen Nylonobjektträger;
eine lichtdurchlässige, optisch klare, kolloidale Nitrocelluloselage, die an der Oberfläche des Objektträgers befestigt ist; und
eine Vielzahl von Sonden, die an die Oberfläche der lichtdurchlässigen, optisch klaren, kolloidalen Nitrozelluloselage gebunden sind, wobei die lichtdurchlässige, optisch klare, kolloidale Nitrozelluloselage eine Bindekapazität von 100-200 µg/cm² hat.

## Revendications

1. Une méthode pour produire des réseaux de matrices translucides **caractérisée par** :
a) dans un premier stade, revêtement d'un substrat avec une couche translucide, optiquement vide, de nitrocellulose colloïdale, où la nitrocellulose colloïdale est en suspension, soit dans de l'acétate d'amyle, soit dans d'autres solvants organiques volatiles, avant application sur le substrat, qui se présente sous la forme d'une lamelle en verre ou autre matériau translucide,
et
b) dans un second stade, fixation d'une ou plusieurs premières sondes sur la couche translucide, optiquement vide, de nitrocellulose colloïdale, où la couche translucide, optiquement vide, de nitrocellulose présente une capacité de liaison aux protéines de 100-200 µg/cm².

2. La méthode de la revendication 1, où le substrat est en verre ou en nylon.

3. La méthode de la revendication 1, où les premières sondes sont sélectionnées dans le groupe comportant anticorps, fragments d'anticorps, fragments F Ab, anticorps humanisés, anticorps simple chaîne, anticorps chimériques, oligonucléotides, polynucléotides, acides nucléiques, aptamères et « affibodies ».

4. La méthode de la revendication 1, où le réseau de matrices peut être reconfiguré.

5. La méthode de la revendication 4, où les premières sondes sont des aptamères ou des « affibodies ».

6. La méthode de la revendication 5, où les premières sondes se lient à IgG (immunoglobuline G).

7. Un dispositif de dosage optique qui peut être obtenu par la méthode de l'une des revendications 1 à 6, comprenant :
une lamelle translucide en nylon ;
une couche translucide, optiquement vide, de nitrocellulose colloïdale fixée sur la surface supérieure de la lamelle ; et
une multiplicité de sondes fixées sur la surface supérieure de la lamelle translucide,
optiquement vide, de nitrocellulose colloïdale, où la couche translucide, optiquement vide,
de nitrocellulose colloïdale a une capacité de liaison de 100-200 µg/cm².
